# EUROPEAN PATENT APPLICATION

(11) **EP 4 234 570 A2**
(43) Date of publication of application: **30.08.2023**
(21) Application number: 23162123.6
(22) Date of filing: 18.09.2019
(51) Int. Cl.: C07K 14/05, C12N 9/22

(54) **PROGRAMMED CELL DEATH 1 (PD1) SPECIFIC NUCLEASES**

(30) Priority: 18.09.2018 US 201862732674 P
(62) Divisional of application: 19862476.9
(71) Applicant: Sangamo Therapeutics, Inc., Brisbane, CA 94005 (US)
(72) Inventor: MILLER, Jeffrey C., Brisbane, 94005 (US); REBAR, Edward J., Brisbane, 94005 (US); ZHANG, Lei, Brisbane, 94005 (US)
(74) Representative: Carpmaels & Ransford LLP

(57) **Abstract**

Described herein are engineered nucleases specific for PD1 gene target sites, the nucleases comprising mutations in the cleavage domain (*e.g., Fok*I or homologue thereof) and/or DNA binding domain (zinc finger protein, TALE, single guide RNA) such that on-target specificity for PD1 gene target sites is increased.

## Description

### CROSS-REFERENCE TO RELATED APPLICATIONS

The present application claims the benefit of U.S. Provisional Application No. 62/732,674, filed September 18, 2018, the disclosure of which is hereby incorporated by reference in its entirety.

### STATEMENT OF RIGHTS TO INVENTIONS

### MADE UNDER FEDERALLY SPONSORED RESEARCH

Not applicable.

### SEQUENCE LISTING

The instant application contains a Sequence Listing which has been submitted electronically in ASCII format and is hereby incorporated by reference in its entirety. Said ASCII copy, created on September 9, 2019, is named 8325018140SL.txt and is 15,792 bytes in size.

### TECHNICAL FIELD

The present disclosure is in the fields of polypeptide and genome engineering and homologous recombination.

### BACKGROUND

Artificial nucleases, such as engineered zinc finger nucleases (ZFN), transcription-activator like effector nucleases (TALENs), the CRISPR/Cas system with an engineered crRNA/tracr RNA ('single guide RNA'), also referred to as RNA guided nucleases, and/or nucleases based on the Argonaute system (*e*.*g*., from *T. thermophilus,* known as 'TtAgo', (Swarts et al (2014) Nature 507(7491): 258-261), comprise DNA binding domains (nucleotide or polypeptide) associated with or operably linked to cleavage domains, and have been used for targeted alteration of genomic sequences. For example, nucleases have been used to insert exogenous sequences, inactivate one or more endogenous genes, create organisms (*e*.*g*., crops) and cell lines with altered gene expression patterns, and the like. See, *e*.*g*., U.S. Patent Nos.9,255,250; 9,200,266; 9,045,763; 9,005,973; 8,956,828; 8,945,868; 8,703,489; 8,586,526; 6,534,261; 6,599,692; 6,503,717; 6,689,558; 7,067,317; 7,262,054; 7,888,121; 7,972,854; 7,914,796; 7,951,925; 8,110,379; 8,409,861; U.S. Patent Publications 20030232410; 20050208489; 20050026157; 20050064474; 20060063231; 20080159996; 201000218264; 20120017290; 20110265198; 20130137104; 20130122591; 20130177983 and 20130177960 and 20150056705. For instance, a pair of nucleases (*e*.*g*., zinc finger nucleases, TALENs, dCas-*Fok* fusions) may be used to cleave genomic sequences. Each member of the pair generally includes an engineered (non-naturally occurring) DNA-binding protein linked to one or more cleavage domains (or half-domains) of a nuclease. When the DNA-binding proteins bind to their target sites, the cleavage domains that are linked to those DNA binding proteins are positioned such that dimerization and subsequent cleavage of the genome can occur.

A number of strategies have been described for enhancing DNA cleavage specificity (reducing off-target cleavage). The details of these approaches have varied with the functional features and limitations of the nucleases to which they have been applied. For all-protein systems (*e*.*g*. TALENs (Miller 2011), ZFNs (Kim 1996), and meganucleases (Grizot 2009)) a simple and direct approach for improving specificity has been to re-engineer the base-sensing interface (Miller 2015; Rebar 1994; Jarjour 2009). Other, more general strategies have included breaking dimerization symmetry to suppress formation of unintended nuclease species (Miller 2007), as well as removing nonspecific DNA contacts (Guilinger 2014). For CRISPR-Cas systems, which feature shorter targets, a number of studies have focused on extending the recognition event required for cleavage to remedy limitations with unique addressing in complex genomes (Ran 2013, Tsai 2014, Guilinger 2014a, Bolukbasi 2015). Opportunities for engineering the base-sensing interface of CRISPR-Cas have been comparatively limited, given the dominant role of Watson-Crick interactions in target recognition, although recent studies have begun to examine the PAM interface and synthetic guides (Yin 2018; Ryan 2018). The specificity of Cas9 has also been improved via removal of nonspecific contacts (Kleinstiver 2016; Slaymaker 2016; Chen 2017) albeit at the cost of reduced activity for at least a subset of the resulting variants (Kulcsar 2017; DeWitt 2016; Zhang 2017). Finally, two recent studies have used selection-based approaches to identify Cas9 variants with greater on-target preference (Hu 2018; Casini 2018). Each of these approaches has yielded reagents with improved specificity relative to a parental starting framework.

A shared feature of these strategies has been their almost exclusive focus on initial target binding for interpretation and rationale. With rare exception, each has openly or implicitly treated target binding as the sole determination of DNA cleavage specificity and as a consequence the potential for improving cleavage preference by re-engineering downstream steps has remained largely unexplored. This would appear to present an important untapped dimension for improving specificity, given the precedence across biology of kinetic modulation for enforcing enzyme specificity as well as the minimal catalytic requirements for the genome editing process (successful editing can require just one cleavage event per cell). Although recent studies of S. *pyogenes* Cas9 have begun to characterize intermediate structures (Singh 2016, Jiang 2016, Jiang 2017) and dynamics of the cleavage process (Sternberg 2015, Dagdas 2017, Raper 2018), efforts to capitalize on these insights have thus far been modest and have not demonstrated unique specificity in highly modified cells (Chen 2017). Moreover, a kinetics-focused framework has not been applied to nucleases such as TALENs and ZFNs, whose longer targets afford an inherently wider energetic gap between on-target and off-target sites where kinetic optimization could be especially beneficial.

U.S. Publication No. 20180087072 discloses highly specific nucleases comprising mutations in the cleavage domain (*e.g., Fok*I) and/or the ZFP backbone and methods of making and using such highly specific nucleases.

The programmed death receptor (PD1, also known as PDCD1) is an immune checkpoint that guards against autoimmunity through two mechanisms: (1) by promoting apoptosis (programmed cell death) of antigen-specific T-cells in lymph nodes; and (2) by reduc apoptosis in regulatory T cells (anti-inflammatory, suppressive T cells). Francisco et al. (2010) Immunological Reviews 236:219-42; Fife et al. (2011) Annals New York Academy of Sciences 1217:45-59. PD-1 inhibitors, a new class of drugs that block PD-1, activate the immune system to attack tumors and are used to treat various types of cancer. *See, e.g.,* Jelinek et al. (2017) Immunology 152(3):357-371.

Thus, there remains a need for improved nucleases targeted to PD1 that exhibit very low or no off-target cleavage activity.

### SUMMARY

The present disclosure provides highly specific PD1 nucleases, namely artificial nucleases (e.g., zinc finger nucleases (ZFNs), TALENs, CRISPR/Cas nucleases) comprising mutations in one or more of the DNA binding domain regions (*e.g.,* the backbone of a zinc finger protein or TALE) and/or one or more mutations in a *FokI* nuclease cleavage domain or cleavage half domain.

Thus, in one aspect, described herein is an engineered (artificial) nuclease targeted to PD1, the nuclease comprising at least one DNA-binding domain (*e.g.,* ZFP) that binds to a target site in a PD1 and at least one cleavage half domain comprising one or more mutations as compared to a parental (*e*.*g*., wild-type) cleavage domain from which these mutants are derived. In certain embodiments, the nuclease is a zinc finger nuclease (ZFN) comprising a pair of ZFNs, also referred to as left and right (or first and second) ZFNs, in which each ZFN of the pair comprises a ZFP PD1 DNA-binding domain and a cleavage domain (or cleavage half-domain). In certain aspects, the PD1 nuclease is a ZFN comprising ZFPs designated 12942 (SEQ ID NO:3) and 25029 (SEQ ID NO:5) and a FokI cleavage domain containing one or more mutations therein. In certain embodiments, the one or more mutations are one or more of the mutations shown in any of the appended Tables and Figures, including any combination of these mutants with each other and with other mutants (such as dimerization and/or catalytic domain mutants as well as nickase mutations). Mutations as described herein, include but are not limited to, mutations that change the charge of the cleavage domain, for example mutations of positively charged residues to non-positively charged residues (*e*.*g*., mutations of K and R residues (e.g., mutated to S); N residues (*e.g.,* to D), and Q residues (*e.g.,* to E); mutations to residues that are predicted to be close to the DNA backbone (*e*.*g*., position (-5), (-3), etc.); and/or mutations at other residues (*e*.*g*., in the dimerization domain).

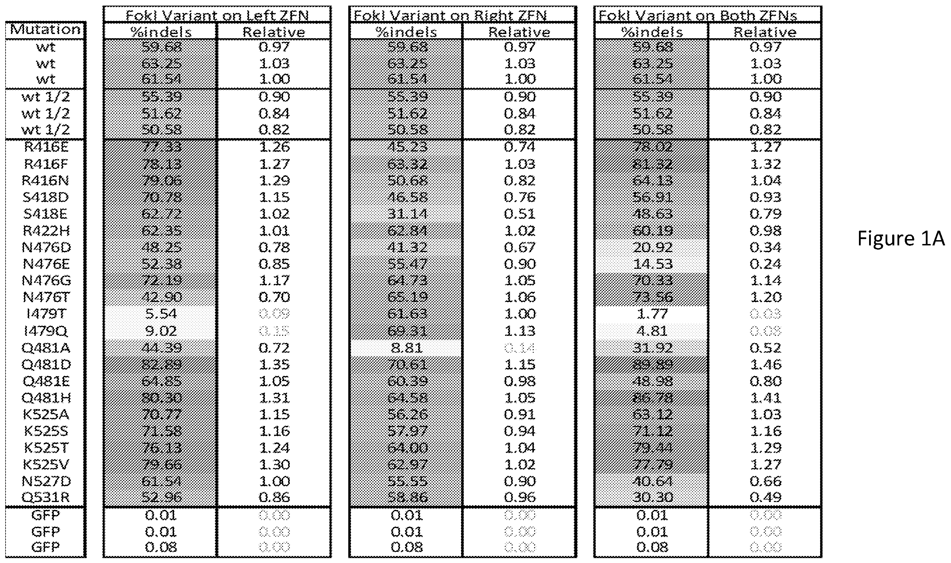

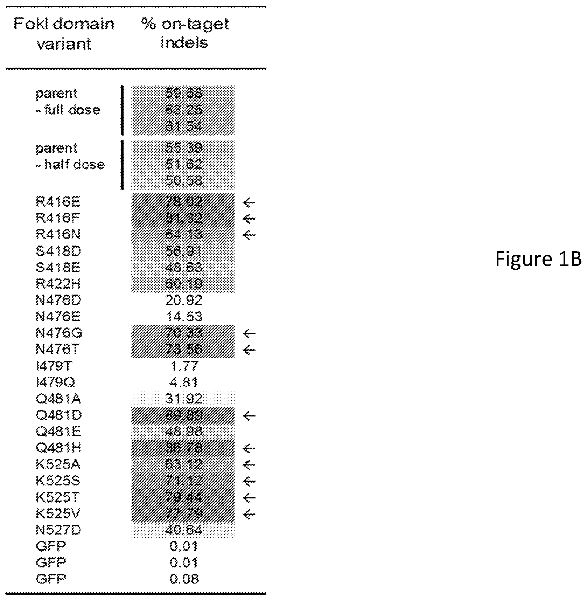

In certain embodiments, the engineered cleavage half domains are derived from *Fok*I or *Fok*I homologues and comprise a mutation in one or more of amino acid residues 414-426, 443-450, 467-488, 501-502, and/or 521-531, including one or more of 387, 393, 394, 398, 400, 416, 418, 422, 427, 434, 439, 441, 442, 444, 446, 448, 472, 473, 476, 478, 479, 480, 481, 487, 495, 497, 506, 516, 523, 525, 527, 529, 534, 559, 569, 570, and/or 571. In certain embodiments, the left ZFN of the ZFN pair comprises the mutation(s). In other embodiments, the right ZFN of the ZFN pair comprises the mutation(s). In other embodiments, both the right and left ZFNs of the ZFN comprise the mutation(s). The mutations may include mutations to residues found in natural restriction enzymes homologous to *Fok*I at the corresponding positions . In certain embodiments, the mutations are substitutions, for example substitution of the wild-type residue with any different amino acid, for example alanine (A), cysteine (C), aspartic acid (D), glutamic acid (E), histidine (H), phenylalanine (F), glycine (G), asparagine (N), serine (S), valine (V), arginine (R), glutamine (Q) or threonine (T). Any combination of mutants is contemplated, including but not limited to those shown in the appended Tables and Figures. In certain embodiments, the *Fok*I nuclease (cleavage) domain comprises a mutation at one or more of 416, 418, 422, 476, 479, 481, 525 and/or 531 (alone or in combination with other mutations such as ELD, KKR, etc.), preferably at 416, 422, 476, 481, and/or 525 and even more preferably at 416, 481 and/or 525. In certain embodiments, at least one *Fok*I domain of the nuclease comprises (i) a single mutation, for example at position 416 (*e.g.,* in which the wild-type R residue is substituted with an E, F, or N residue), at position 418 (*e.g.,* in which the wild- type S residue is substitute with a D or E residue), at position 422 (*e*.*g*., in which the wild-type R residue is substituted with an H residue), at position 476 (*e*.*g*., in which the wild-type N residue is substituted with a D, E, G or T residue), at position 481 (*e.g.,* in which the wild-type Q residue is substituted with a D, E or H residue), at position 525 (*e*.*g*., in which the wild-type K residue is substituted with an A, S, T or V residue), at position 527 (*e.g.,* in which the wild-type N residue is substituted with a D residue), or at position 531 (*e.g.,* in which the wild-type Q residue is substituted with an R residue); or (ii) a mutation at one of the 416 (*e*.*g*., in which the wild-type R residue is substituted with an E, F, or N residue), at position 418 (*e.g.,* in which the wild- type S residue is substitute with a D or E residue), at position 422 (*e.g.,* in which the wild-type R residue is substituted with an H residue), at position 476 (*e*.*g*., in which the wild-type N residue is substituted with a D, E, G or T residue), at position 481 (*e.g.,* in which the wild-type Q residue is substituted with a D, E or H residue), at position 525 (*e.g.,* in which the wild-type K residue is substituted with an A, S, T or V residue), at position 527 (*e*.*g*., in which the wild-type N residue is substituted with a D residue), or at position 531 (*e*.*g*., in which the wild-type Q residue is substituted with an R residue) in combination with mutations one or more additional residues (e.g., dimerization mutations such as ELD, KKR, etc.). *See, e.g.,* Figures 1-3. In certain embodiments, the mutation comprises a single mutation selected from the group consisting of: R416E, R416F, R416N, S418D, S418E, R422H, N476D, N476E, N476G, N476T, I479T, I479Q, Q481A, Q481D, Q481E, Q481H, K525A, K525S, K525T, K525V, N527D, and/or Q531R mutations. Non-limiting examples of substitution mutations are shown in Figures 1-3. The nuclease (cleavage) domains of one or both components of a nuclease pair may also comprise one or more mutations at positions 418, 432, 441, 448, 476, 481, 483, 486, 487, 490, 496, 499, 523, 527, 537, 538 and 559, including but not limited to ELD, KKR, ELE, KKS. *See, e.g.,* U.S. Patent No. 8,623,618.

Thus, described herein is a zinc finger nuclease (ZFN) or TALEN that cleaves a programmed cell death 1 (PD1) gene, the ZFN or TALEN comprising first and second (also referred to as left and right) ZFNs and TALENs, each ZFN comprising a ZFP DNA-binding domain that binds to a target site in the PD1 gene and a *Fok*I cleavage domain, each TALEN comprising a TAL-effector DNA-binding domain that binds to a target site in the PD1 gene and a *Fok*I cleavage domain, wherein at least one of the *FokI* cleavage domains of the ZFN or TALEN further comprises a substitution mutation in the *Fok*I cleavage domain at one or more of 416, 418, 422, 476, 479, 481, 525, 527 or 531, numbered relative to wild-type *Fok*I*.* In certain embodiments, the substitution mutation in the first and/or second *Fok*I cleavage domain is as follows: R416E, R416F, R416N, S418D, S418E, R422H, N476D, N476E, N476G, N476T, I479T, I479Q, Q481A, Q481D, Q481E, Q481H, K525A, K525S, K525T, K525V, N527D and/or Q531R. In certain embodiments, the ZFN or TALEN of any of the preceding claims, wherein the substitution mutation in the first and/or second *Fok*I cleavage domain is as follows: R416E, R416F, R416N, R422H, N476G, N476T, Q481D, Q481H, K525A, K525S, K525T or K525V. In certain embodiments, the nuclease comprises a first ZFN having the amino acid sequence as shown in SEQ ID NO:3 and a second ZFP DNA-binding domain comprises the ZFP having the amino acid sequence as shown in SEQ ID NO:5.

The artificial nucleases described herein may further include mutations to one or more amino acids within the DNA binding domain outside the residues that recognize the nucleotides of the target sequence (e.g., one or more mutations to the 'ZFP backbone' (outside the DNA recognition helix region) or to the `TALE backbone' (outside of the RVDs)) that can interact non-specifically with phosphates on the DNA backbone. Thus, in certain embodiments, the invention includes mutations of cationic amino acid residues in the ZFP backbone that are not required for nucleotide target specificity. In some embodiments, these mutations in the ZFP backbone comprise mutating a cationic amino acid residue to a neutral or anionic amino acid residue. In some embodiments, these mutations in the ZFP backbone comprise mutating a polar amino acid residue to a neutral or non-polar amino acid residue. In preferred embodiments, mutations are made at position (-5), (-9) and/or position (-14) relative to the DNA binding helix. In some embodiments, a zinc finger may comprise one or more mutations at (-5), (-9) and/or (-14). In further embodiments, one or more zinc fingers in a multi-finger zinc finger protein may comprise mutations in (-5), (-9) and/or (-14). In certain embodiments, 1, 2, 3, 4, 5 or 6 of the fingers of a zinc finger protein comprise one or more backbone mutations (e.g., (-5) in 1, 2, 3, 4, 5 or 6 of the fingers). In some embodiments, the amino acids at (-5), (-9) and/or (-14) (*e.g.* an arginine (R) or lysine (K)) are mutated to an alanine (A), leucine (L), Ser (S), Asp (N), Glu (E), Tyr (Y) and/or glutamine (Q). In some embodiments, the Arg (R) at position -5 is changed to a Tyr (Y), Asp (N), Glu (E), Leu (L), Gln (Q), or Ala (A). In other embodiments, the Arg (R) at position (-9) is replaced with Ser (S), Asp (N), or Glu (E). In further embodiments, the Arg (R) at position (-14) is replaced with Ser (S) or Gln (Q). In other embodiments, the fusion polypeptides can comprise mutations in the zinc finger DNA binding domain where the amino acids at the (-5), (-9) and/or (-14) positions are changed in one or more fingers (*e.g.,* 3 fingers, 4 fingers, 5 fingers of 6 fingers of a ZFP) to any of the above listed amino acids in any combination.

The mutations described herein (alone or in any combination) provide PD1 nucleases with increased specificity.

In another aspect, polynucleotides encoding any of the engineered cleavage half-domains or fusion proteins as described herein are provided. Thus, one or more polynucleotides encoding one or more ZFNs or TALENs are also provided.

In yet another aspect, cells comprising any of the nucleases, polypeptides (e.g., fusion molecules or fusion polypeptides) and/or polynucleotides as described herein are also provided, including isolated cells (or populations of cells) comprising one or more ZFNs, one or more TALENs and/or one or more polynucleotides as described herein. Also provided is an isolated population of genetically modified cells produced from the isolated cell or methods as described herein, wherein PD1 gene is specifically modified by the nucleases. In certain embodiments, the on target to off-target ratio of genetic modification of PD1 is greater than 200 in the isolated population of cells. Also described is a partially or fully differentiated cell descended from the isolated population of genetically modified cells as described herein. The isolated population of genetically modified cells as described herein may further comprise one or more additional genetic modifications, optionally comprising inactivating one or more genes other than PD1 such as a T cell receptor gene, a B2M gene and/or a CTLA-4 gene, and/or integration of a transgene such as a CAR transgene.

In one embodiment, the cells comprise a pair of fusion polypeptides, one or both fusion polypeptides comprising, in addition to one or more mutations as described herein, one or more additional mutations at residues for example engineered cleavage half-domain as described in U.S. Patent 8,962,281.

Also provided herein are cells that have been modified by the polypeptides and/or polynucleotides of the invention. In some embodiments, the cells comprise a nuclease-mediated insertion of a transgene, or a nuclease-mediated knock out of a PD1 gene. The modified cells, and any cells derived from the modified cells do not necessarily comprise the nucleases of the invention more than transiently, but the genomic modifications mediated by such nucleases remain.

In yet another aspect, methods for targeted cleavage of a PD1 gene; methods of causing homologous recombination to occur in a cell; methods of treating infection; and/or methods of treating disease are provided. These methods maybe practiced *in vitro, ex vivo* or *in vivo* or a combination thereof. The methods involve cleaving cellular chromatin at a predetermined region of interest in cells by expressing a pair of fusion polypeptides as described herein (*i*.*e*., a pair of fusion polypeptides in which one or both fusion polypeptide(s) comprises the engineered cleavage half-domains as described herein). In certain embodiments, the targeted cleavage preference for the on-target site (*e.g.,* PD1 gene) over off-target sites (*e.g.,* non-PD1 gene) is increased by at least 50 to 200% (or any value therebetween) or more, including 50%-60% (or any value therebetween), 60%-70% (or any value therebetween), 70%-80% (or any value therebetween), 80%-90% (or any value therebetween), 90% to 200% (or any value therebetween), or any value > 200%, as compared to nucleases comprising cleavage domains without the mutations as described herein. Similarly, using the methods and compositions as described herein, off-target site cleavage is reduced by 1-100 or more-fold, including but not limited to 1-50-fold (or any value therebetween). In other aspects, the components (left and right) of a paired PD1 nuclease as described herein are administered separately in any ratio. In some embodiments, the left and right components are administered in equal quantities. In some embodiments, the engineered cleavage half-domain partners of an engineered nuclease complex are used to contact a cell, where each partner of the complex is given at a 1:1 ratio, or in a ratio to the other partner other than one to one. In some embodiments, the ratio of the two partners (half cleavage domains) is given at a 1:1, 1:2, 1:3, 1:4, 1:5, 1:6, 1:8, 1:9, 1:10 or 1:20 ratio, or any value therebetween. In other embodiments, the ratio of the two partners is greater than 1:30. In other embodiments, the two partners are deployed at a ratio that is chosen to be different from 1:1. In some aspects, each partner is delivered to the cell as an mRNA or is delivered in a viral or non-viral vector where equal or non-equal quantities of mRNA or vector encoding each partner are delivered. In further embodiments, each partner of the nuclease complex may be comprised on a single viral or non-viral vector, but is deliberately expressed such that both partners are expressed equally, or one partner is expressed at a higher or lower value that the other, ultimately delivering the cell a ratio of cleavage half domains that is other than one to one. In some embodiments, each cleavage half domain is expressed using different promoters with different expression efficiencies. In other embodiments, the two cleavage domains are delivered to the cell using a viral or non-viral vector where both are expressed from the same open reading frame, but the genes encoding the two partners are separated by a sequence (e.g. self-cleaving 2A sequence or IRES) that results in the 3' partner being expressed at a lower rate, such that the ratios of the two partners are 1:2, 1:3, 1:4, 1:5, 1:6, 1:8, 1:9, 1:10 or 1:20 ratio, or any value therebetween. In other embodiments, the two partners are deployed equal ratios, or at a ratio that is chosen to be different from 1:1.

Also provided are methods of altering an endogenous PD1 gene, for example to introduce targeted mutations. In certain embodiments, methods of genetically modifying a PD1 gene include introducing into the cell one or more targeted nucleases to create a double-stranded break in cellular chromatin at a predetermined site, and a donor polynucleotide, having homology to the nucleotide sequence of the cellular chromatin in the region of the break. Cellular DNA repair processes are activated by the presence of the double-stranded break and the donor polynucleotide is used as a template for repair of the break, resulting in the introduction of all or part of the nucleotide sequence of the donor into the cellular chromatin. Thus, a PD1 sequence in cellular chromatin can be altered and, in certain embodiments, can be converted into a sequence present in a donor polynucleotide.

Targeted alterations include, but are not limited to, point mutations *(i.e.,* conversion of a single base pair to a different base pair), substitutions (*i.e.*, conversion of a plurality of base pairs to a different sequence of identical length), insertions or one or more base pairs, deletions of one or more base pairs and any combination of the aforementioned sequence alterations. Alterations can also include conversion of base pairs that are part of a coding sequence such that the encoded amino acid is altered.

The donor polynucleotide can be DNA or RNA, can be linear or circular, and can be single-stranded or double-stranded. It can be delivered to the cell as naked nucleic acid, as a complex with one or more delivery agents (*e*.*g*., liposomes, nanoparticles, poloxamers) or contained in a viral delivery vehicle, such as, for example, an adenovirus, lentivirus or an Adeno-Associated Virus (AAV). Donor sequences can range in length from 10 to 5,000 nucleotides (or any integral value of nucleotides therebetween) or longer. In some embodiments, the donor comprises a full-length gene flanked by regions of homology with the targeted cleavage site. In some embodiments, the donor lacks homologous regions and is integrated into a target locus through homology independent mechanism (*i.e.* NHEJ). In other embodiments, the donor comprises a smaller piece of nucleic acid flanked by homologous regions for use in the cell (*i.e.* for gene correction). In some embodiments, the donor comprises a gene encoding a functional or structural component such as a shRNA, RNAi, miRNA or the like. In other embodiments, the donor comprises sequences encoding a regulatory element that binds to and/or modulates expression of a gene of interest. In other embodiments, the donor is a regulatory protein of interest (*e*.*g*. ZFP TFs, TALE TFs or a CRISPR/Cas TF) that binds to and/or modulates expression of a gene of interest.

In yet another aspect, cells comprising any of the polypeptides (*e*.*g*., fusion molecules) and/or polynucleotides as described herein are also provided. In one embodiment, the cells comprise a pair of fusion molecules, each comprising a cleavage domain as disclosed herein. Also provided herein is an isolated population of genetically modified cells (*e*.*g*., T cells) produced using the nuclease(s) as described herein, wherein the PD1 gene is specifically modified (as compared to other genes) by the nucleases. In these cells, the PD1 gene in these cells is genetically modified (*e*.*g*., mutated by insertions and/or deletions ("indels")) by the nuclease(s) but genetic modifications outside of the PD1 gene made using these nucleases are reduced by 1-100 or more-fold, including but not limited to 1-50-fold (or any value therebetween), as compared to PD1 nucleases without *Fok*I mutation(s) described herein. In certain embodiments, less than 1% (*e.g.,* less than 0.5%) of the genetic modifications made by the nuclease(s) in the isolated population of cells are outside of the PD1 gene. In certain aspects, at least 40% of the cells of the population produced by the nuclease include modifications (indels) to PD1 while less than 0.05% of the cells include off-target (non-PD1) genetic modifications made by the nucleases. In still further embodiments, the isolated population of genetically modified cells produced using the nucleases described herein have a greater relative on/off (PD1/non-PD1) ratio of genetic modification as compared to the on/off ratio of genetic modification using PD1 targeted nucleases without the *FokI* mutations described herein, optionally wherein the on/off genetic modification ratio in the cells made by the nucleases of the invention is 100 or more, or 150 or more, or 200 or more, as shown in the appended Figures. Cells include cultured cells, cells in an organism and cells that have been removed from an organism for treatment in cases where the cells and/or their descendants will be returned to the organism after treatment. A region of interest in cellular chromatin can be, for example, a genomic sequence or portion thereof.

A composition comprising one or more ZFNs or TALENs, one or more polynucleotides or the isolated population of cells as described herein claims for use in treatment of a disease or disorder such as a cancer. Also provided is a method of treating a disease or disorder (*e*.*g*., a cancer) in a subject, the method comprising cleaving a PD1 gene administering one or more ZFNs or TALENs, one or more polynucleotides or the isolated population of cells as described herein to a subject in need thereof.

In another aspect, described herein is a kit comprising a fusion protein as described herein or a polynucleotide encoding one or more zinc finger proteins, cleavage domains and/or fusion proteins as described herein; ancillary reagents; and optionally instructions and suitable containers. The kit may also include one or more nucleases or polynucleotides encoding such nucleases.

These and other aspects will be readily apparent to the skilled artisan in light of the disclosure as a whole.

### BRIEF DESCRIPTION OF THE DRAWINGS

**Figure 1A** **and** **Figure 1B** show results *of Fok*I variant screening data for the indicated PD1 ZFN pair. **Figure 1A** shows results of the indicated variants. Values were determined from a single replicate with a dose of 500 ng mRNA for each ZFN in the pair. "wt" indicates the previously reported PD1 ZFN pair (*e*.*g*., 12942/25029). "wt ½" indicates the previously reported PD1 ZFN pair tested at a dose of 250 ng of each ZFN monomer. Relative indicates the %indels as a fraction of the average of the three replicate measurements for the "wt" sample. The left two columns of values were determined with the indicated *Fok*I*.* in the left ZFN combined with the previously reported right ZFN. The middle columns of values were similar except that the indicated *Fok*I variant is on the right ZFN and the left ZFN is unmodified. The rightmost columns of values were determined with the indicated *Fok*I variant on both the left and right ZFN. **Figure 1B** shows on-target activity of variants of the PD1 ZFN dimer bearing single-residue substitutions within their *Fok*I domain as indicated. "Half dose" parent samples used 250 ng RNA for delivery. To highlight relative signal intensities table values are embedded in a gray heat map. Arrows highlight variants manifesting full retention of high levels of on-target activity that were further characterized in follow-up studies.

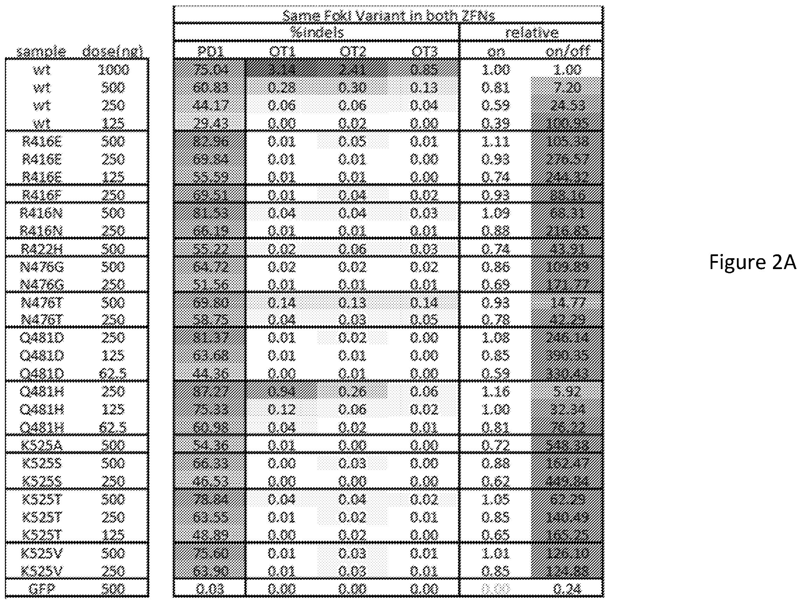

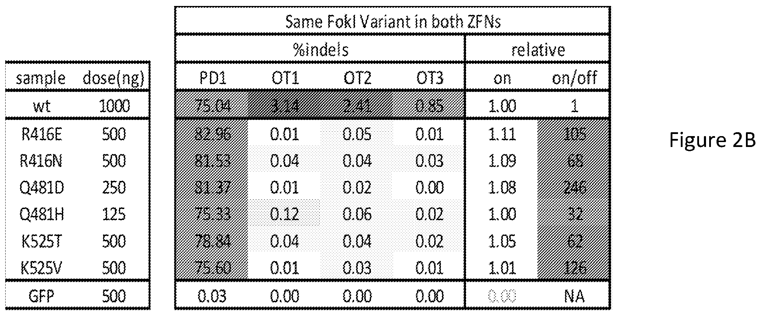

**Figure 2A** **and** **Figure 2B** are tables showing on target ("PD1") and off-target ("OT1" "OT2" and "OT3") cleavage activity (% indels) of the indicated *FokI* variants for the PD1 ZFN pair tested at multiple doses. **Figure 2A** shows results for R416E, R416F, R416N, R422H, N476G, N476T, Q481D, Q481H, K525A, K525S, K525T, K525V (and GFP control). **Figure 2B** shows a subset of the data shown in Figure 2A (R416E, R46N, Q481D, Q481H, K525T and K525V). Values are the average of three biologic replicates. "wt" indicates the previously reported PD1 ZFN pair (12942/25029). For other samples, the indicated *Fok*I variant in the left column was used on both the left and right ZFN. The mRNA dose of each ZFN in the pair is indicated in the second column. Also shown is the relative cleavage activity as between on target and off-target sites (last column "on/off").

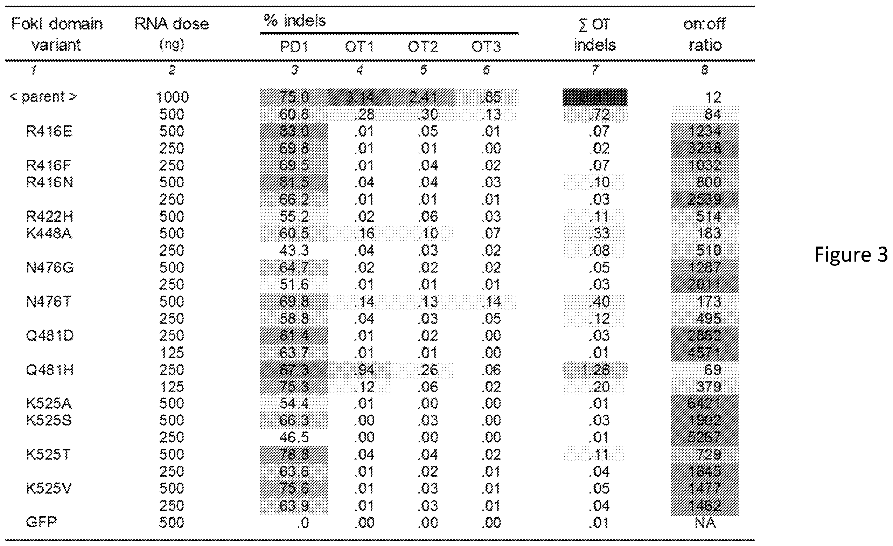

**Figure 3** shows on target and off-target activity of variants of the PD1 ZFN dimer bearing single-residue substitutions within their *Fok*I domain. Each variant was tested as a dimer in which both ZFNs bore the indicated substitution (column 1, "parent" indicates pair prior to making the indicated *Fok*I mutations (*e.g.,* 12942/25029). ZFNs were delivered to human K562 cells via nucleofection using the indicated amount of mRNA for each ZFN monomer (column 2), followed by genomic DNA isolation at 3 days and deep sequencing analysis for indels at the intended target. Column 3 provides % indels measured at the intended target, with columns 4-6 indicating % indels at three previously known off-target sites. Values are the average of three biological replicates. Column 7 lists the sum of off-target indels, with column 8 showing on target:off-target indel ratio (= column 3 / column 7).

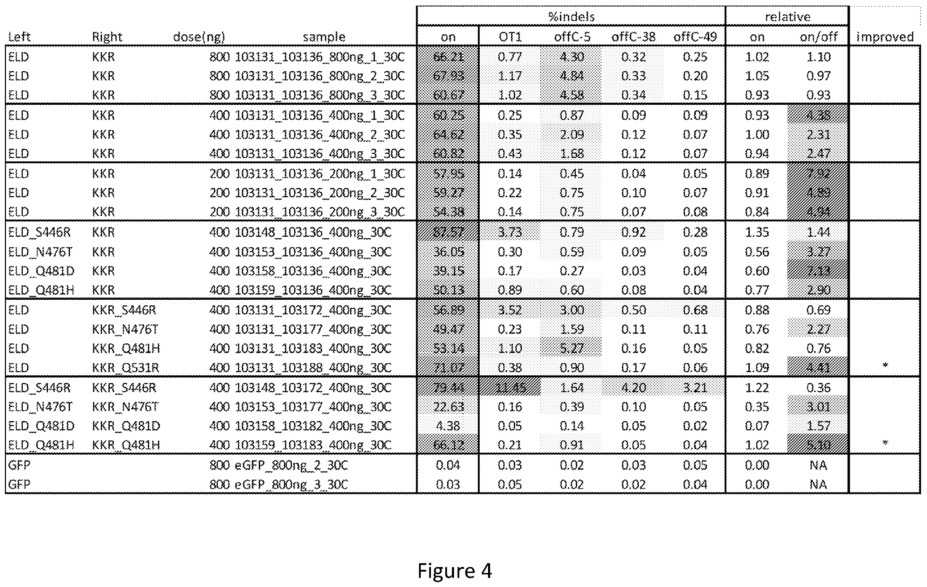

**Figure 4** shows results of cleavage using the indicated TALEN pairs (targeted to CCR5) at on and off-target sites at the indicated dosages. Each variant was tested as a dimer in which both TALENs bore the indicated substitution (column 1, "parent" indicates the CCR5 TALEN pair without the indicated *Fok*I mutations). TALENs were delivered to human K562 cells via nucleofection using the indicated amount of mRNA for each ZFN monomer (column 2), followed by genomic DNA isolation at 3 days and deep sequencing analysis for indels at the intended target. Column 3 provides % indels measured at the intended target, with columns 4-6 indicating % indels at three previously known off-target sites. Values are the average of three biological replicates. Column 7 lists the sum of off-target indels, with column 8 giving on target off-target indel ratio (= column 3 / column 7). To help highlight relative signal intensities, table values are embedded in gray heat maps.

### DETAILED DESCRIPTION

Disclosed herein are methods and compositions for increasing specificity of on-target engineered nuclease cleavage of a PD1 gene via differentially decreasing off-target cleavage.

### General

Practice of the methods, as well as preparation and use of the compositions disclosed herein employ, unless otherwise indicated, conventional techniques in molecular biology, biochemistry, chromatin structure and analysis, computational chemistry, cell culture, recombinant DNA and related fields as are within the skill of the art. These techniques are fully explained in the literature. *See,* for example, Sambrook et al. MOLECULAR CLONING: A LABORATORY MANUAL, Second edition, Cold Spring Harbor Laboratory Press, 1989 and Third edition, 2001; Ausubel et al., CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, "Chromatin" (P.M. Wassarman and A. P. Wolffe, eds.), Academic Press, San Diego, 1999; and METHODS IN MOLECULAR BIOLOGY, Vol. 119, "Chromatin Protocols" (P.B. Becker, ed.) Humana Press, Totowa, 1999.

### Definitions

The terms "nucleic acid," "polynucleotide," and "oligonucleotide" are used interchangeably and refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogues of natural nucleotides, as well as nucleotides that are modified in the base, sugar and/or phosphate moieties (*e*.*g*., phosphorothioate backbones). In general, an analogue of a particular nucleotide has the same base-pairing specificity; *i*.*e*., an analogue of A will base-pair with T.

The terms "polypeptide," "peptide" and "protein" are used interchangeably to refer to a polymer of amino acid residues. The term also applies to amino acid polymers in which one or more amino acids are chemical analogues or modified derivatives of a corresponding naturally-occurring amino acids.

"Binding" refers to a sequence-specific, non-covalent interaction between macromolecules (*e*.*g*., between a protein and a nucleic acid). Not all components of a binding interaction need be sequence-specific (*e*.*g*., contacts with phosphate residues in a DNA backbone), as long as the interaction as a whole is sequence-specific. Such interactions are generally characterized by a dissociation constant (K_{d}) of 10⁻⁶ M⁻¹ or lower. "Affinity" refers to the strength of binding: increased binding affinity being correlated with a lower K_{d}. "Non-specific binding" refers to, non-covalent interactions that occur between any molecule of interest (*e*.*g*. an engineered nuclease) and a macromolecule (*e*.*g*. DNA) that are not dependent on-target sequence.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, *etc*.) and/or it can bind to one or more molecules of a different protein or proteins. A binding protein can have more than one type of binding activity. For example, zinc finger proteins have DNA-binding, RNA-binding and protein-binding activity. In the case of an RNA-guided nuclease system, the RNA guide is heterologous to the nuclease component (Cas9 or Cfp1) and both may be engineered.

A "DNA binding molecule" is a molecule that can bind to DNA. Such DNA binding molecule can be a polypeptide, a domain of a protein, a domain within a larger protein or a polynucleotide. In some embodiments, the polynucleotide is DNA, while in other embodiments, the polynucleotide is RNA. In some embodiments, the DNA binding molecule is a protein domain of a nuclease (*e*.*g*. the *Fok*I domain), while in other embodiments, the DNA binding molecule is a guide RNA component of an RNA-guided nuclease (e.g. Cas9 or Cfp1).

A "DNA binding protein" (or binding domain) is a protein, or a domain within a larger protein, that binds DNA in a sequence-specific manner, for example through one or more zinc fingers or through interaction with one or more RVDs in a zinc finger protein or TALE, respectively. The term zinc finger DNA binding protein is often abbreviated as zinc finger protein or ZFP.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP. The term "zinc finger nuclease" includes one ZFN as well as a pair of ZFNs (the members of the pair are referred to as "left and right" or "first and second" or "pair") that dimerize to cleave the target gene.

A "TALE DNA binding domain" or "TALE" is a polypeptide comprising one or more TALE repeat domains/units. The repeat domains are involved in binding of the TALE to its cognate target DNA sequence. A single "repeat unit" (also referred to as a "repeat") is typically 33-35 amino acids in length and exhibits at least some sequence homology with other TALE repeat sequences within a naturally occurring TALE protein. *See, e.g.,* U.S. Patent No. 8,586,526, incorporated by reference herein in its entirety. The term "TALEN" includes one TALEN as well as a pair of TALENs (the members of the pair are referred to as "left and right" or "first and second" or "pair") that dimerize to cleave the target gene.

Zinc finger and TALE DNA-binding domains can be "engineered" to bind to a predetermined nucleotide sequence, for example via engineering (altering one or more amino acids) of the recognition helix region of a naturally occurring zinc finger protein or by engineering of the amino acids involved in DNA binding (the "repeat variable diresidue" or RVD region). Therefore, engineered zinc finger proteins or TALE proteins are proteins that are non-naturally occurring. Non-limiting examples of methods for engineering zinc finger proteins and TALEs are design and selection. A designed protein is a protein not occurring in nature whose design/composition results principally from rational criteria. Rational criteria for design include application of substitution rules and computerized algorithms for processing information in a database storing information of existing ZFP or TALE designs and binding data. See, for example, U.S. Patent Nos. 8,586,526; 6,140,081; 6,453,242; and 6,534,261; see also WO 98/53058; WO 98/53059; WO 98/53060; WO 02/016536 and WO 03/016496.

A "selected" zinc finger protein, TALE protein or CRISPR/Cas system is not found in nature whose production results primarily from an empirical process such as phage display, interaction trap, rational design or hybrid selection. See *e.g.,* US 5,789,538; US 5,925,523; US 6,007,988; US 6,013,453; US 6,200,759; WO 95/19431; WO 96/06166; WO 98/53057; WO 98/54311; WO 00/27878; WO 01/60970; WO 01/88197 and WO 02/099084.

"TtAgo" is a prokaryotic Argonaute protein thought to be involved in gene silencing. TtAgo is derived from the bacteria *Thermus thermophilus.* See, *e.g.* Swarts *et al, ibid*; G. Sheng et al., (2013) Proc. Natl. Acad. Sci. U.S.A. 111, 652). A "TtAgo system" is all the components required including e.g. guide DNAs for cleavage by a TtAgo enzyme.

"Recombination" refers to a process of exchange of genetic information between two polynucleotides, including but not limited to, capture by non-homologous end joining (NHEJ) and homologous recombination. For the purposes of this disclosure, "homologous recombination (HR)" refers to the specialized form of such exchange that takes place, for example, during repair of double-strand breaks in cells via homology-directed repair mechanisms. This process requires nucleotide sequence homology, uses a "donor" molecule to template repair of a "target" molecule (*i.e.,* the one that experienced the double-strand break), and is variously known as "non-crossover gene conversion" or "short tract gene conversion," because it leads to the transfer of genetic information from the donor to the target. Without wishing to be bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized HR often results in an alteration of the sequence of the target molecule such that part or all of the sequence of the donor polynucleotide is incorporated into the target polynucleotide.

In certain methods of the disclosure, one or more targeted nucleases as described herein create a double-stranded break (DSB) in the target sequence (*e*.*g*., cellular chromatin) at a predetermined site (*e.g.,* a gene or locus of interest). The DSB mediates integration of a construct (*e*.*g*. donor) as described herein. Optionally, the construct has homology to the nucleotide sequence in the region of the break. An expression construct may be physically integrated or, alternatively, the expression cassette is used as a template for repair of the break via homologous recombination, resulting in the introduction of all or part of the nucleotide sequence as in the expression cassette into the cellular chromatin. Thus, a first sequence in cellular chromatin can be altered and, in certain embodiments, can be converted into a sequence present in an expression cassette. Thus, the use of the terms "replace" or "replacement" can be understood to represent replacement of one nucleotide sequence by another, (*i.e.,* replacement of a sequence in the informational sense), and does not necessarily require physical or chemical replacement of one polynucleotide by another.

In any of the methods and compositions (*e*.*g*., nucleases, cells made using these nucleases, etc.) described herein, additional engineered nucleases can be used for additional double-stranded cleavage of additional target sites within the cell.

In certain embodiments of methods for targeted recombination and/or replacement and/or alteration of a sequence in a region of interest in cellular chromatin, a chromosomal sequence is altered by homologous recombination with an exogenous "donor" nucleotide sequence. Such homologous recombination is stimulated by the presence of a double-stranded break in cellular chromatin, if sequences homologous to the region of the break are present.

In any of the methods and compositions (*e*.*g*., nucleases, cells made using these nucleases, etc.) described herein, the first nucleotide sequence (the "donor sequence") can contain sequences that are homologous, but not identical, to genomic sequences in the region of interest, thereby stimulating homologous recombination to insert a non-identical sequence in the region of interest. Thus, in certain embodiments, portions of the donor sequence that are homologous to sequences in the region of interest exhibit between about 80 to 99% (or any integer therebetween) sequence identity to the genomic sequence that is replaced. In other embodiments, the homology between the donor and genomic sequence is higher than 99%, for example if only 1 nucleotide differs as between donor and genomic sequences of over 100 contiguous base pairs. In certain cases, a non-homologous portion of the donor sequence can contain sequences not present in the region of interest, such that new sequences are introduced into the region of interest. In these instances, the non-homologous sequence is generally flanked by sequences of 50-1,000 base pairs (or any integral value therebetween) or any number of base pairs greater than 1,000, that are homologous or identical to sequences in the region of interest. In other embodiments, the donor sequence is non-homologous to the first sequence, and is inserted into the genome by non-homologous recombination mechanisms.

Any of the methods described herein can be used for partial or complete inactivation of one or more target sequences in a cell by targeted integration of donor sequence or via cleavage of the target sequence(s) followed by error-prone NHEJ-mediated repair that disrupts expression of the gene(s) of interest. Cell lines with partially or completely inactivated genes are also provided.

Furthermore, the methods of targeted integration as described herein can also be used to integrate one or more exogenous sequences. The exogenous nucleic acid sequence can comprise, for example, one or more genes or cDNA molecules, or any type of coding or noncoding sequence, as well as one or more control elements (*e*.*g*., promoters). In addition, the exogenous nucleic acid sequence may produce one or more RNA molecules (*e*.*g*., small hairpin RNAs (shRNAs), inhibitory RNAs (RNAis), microRNAs (miRNAs), *etc*.).

"Cleavage" refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In certain embodiments, fusion polypeptides are used for targeted double-stranded DNA cleavage.

A "cleavage half-domain" is a polypeptide sequence which, in conjunction with a second polypeptide (either identical or different) forms a complex having cleavage activity (preferably double-strand cleavage activity). The terms "first and second cleavage half-domains;" "+ and - cleavage half-domains" and "right and left cleavage half-domains" are used interchangeably to refer to pairs of cleavage half-domains that dimerize. The term "cleavage domain" is used interchangeably with the term "cleavage half-domain." The term *"FokI* cleavage domain" includes the *FokI* sequence as shown in herein as well as any *FokI* homologues.

An "engineered cleavage half-domain" is a cleavage half-domain that has been modified so as to form obligate heterodimers with another cleavage half-domain (*e*.*g*., another engineered cleavage half-domain).

The term "sequence" refers to a nucleotide sequence of any length, which can be DNA or RNA; can be linear, circular or branched and can be either single-stranded or double stranded. The term "transgene" refers to a nucleotide sequence that is inserted into a genome. A transgene can be of any length, for example between 2 and 100,000,000 nucleotides in length (or any integer value therebetween or thereabove), preferably between about 100 and 100,000 nucleotides in length (or any integer therebetween), more preferably between about 2000 and 20,000 nucleotides in length (or any value therebetween) and even more preferable, between about 5 and 15 kb (or any value therebetween).

A "chromosome," is a chromatin complex comprising all or a portion of the genome of a cell. The genome of a cell is often characterized by its karyotype, which is the collection of all the chromosomes that comprise the genome of the cell. The genome of a cell can comprise one or more chromosomes.

An "episome" is a replicating nucleic acid, nucleoprotein complex or other structure comprising a nucleic acid that is not part of the chromosomal karyotype of a cell. Examples of episomes include plasmids, minicircles and certain viral genomes. The liver specific constructs described herein may be episomally maintained or, alternatively, may be stably integrated into the cell.

An "exogenous" molecule is a molecule that is not normally present in a cell, but can be introduced into a cell by one or more genetic, biochemical or other methods. "Normal presence in the cell" is determined with respect to the particular developmental stage and environmental conditions of the cell. Thus, for example, a molecule that is present only during embryonic development of muscle is an exogenous molecule with respect to an adult muscle cell. Similarly, a molecule induced by heat shock is an exogenous molecule with respect to a non-heat-shocked cell. An exogenous molecule can comprise, for example, a functioning version of a malfunctioning endogenous molecule or a malfunctioning version of a normally-functioning endogenous molecule.

An exogenous molecule can be, among other things, a small molecule, such as is generated by a combinatorial chemistry process, or a macromolecule such as a protein, nucleic acid, carbohydrate, lipid, glycoprotein, lipoprotein, polysaccharide, any modified derivative of the above molecules, or any complex comprising one or more of the above molecules. Nucleic acids include DNA and RNA, can be single- or double-stranded; can be linear, branched or circular; and can be of any length. Nucleic acids include those capable of forming duplexes, as well as triplex-forming nucleic acids. See, for example, U.S. Patent Nos. 5,176,996 and 5,422,251. Proteins include, but are not limited to, DNA-binding proteins, transcription factors, chromatin remodeling factors, methylated DNA binding proteins, polymerases, methylases, demethylases, acetylases, deacetylases, kinases, phosphatases, ligases, deubiquitinases, integrases, recombinases, ligases, topoisomerases, gyrases and helicases.

An exogenous molecule can be the same type of molecule as an endogenous molecule, *e*.*g*., an exogenous protein or nucleic acid. For example, an exogenous nucleic acid can comprise an infecting viral genome, a plasmid or episome introduced into a cell, or a chromosome that is not normally present in the cell. Methods for the introduction of exogenous molecules into cells are known to those of skill in the art and include, but are not limited to, lipid-mediated transfer (*i.e.*, liposomes, including neutral and cationic lipids), electroporation, direct injection, cell fusion, particle bombardment, calcium phosphate co-precipitation, DEAE-dextran-mediated transfer and viral vector-mediated transfer. An exogenous molecule can also be the same type of molecule as an endogenous molecule but derived from a different species than the cell is derived from. For example, a human nucleic acid sequence may be introduced into a cell line originally derived from a mouse or hamster. Methods for the introduction of exogenous molecules into plant cells are known to those of skill in the art and include, but are not limited to, protoplast transformation, silicon carbide (*e.g.,* WHISKERS^{™}), *Agrobacterium-mediated* transformation, lipid-mediated transfer (*i.e.,* liposomes, including neutral and cationic lipids), electroporation, direct injection, cell fusion, particle bombardment (*e*.*g*., using a "gene gun"), calcium phosphate co-precipitation, DEAE-dextran-mediated transfer and viral vector-mediated transfer.

By contrast, an "endogenous" molecule is one that is normally present in a particular cell at a particular developmental stage under particular environmental conditions. For example, an endogenous nucleic acid can comprise a chromosome, the genome of a mitochondrion, chloroplast or other organelle, or a naturally-occurring episomal nucleic acid. Additional endogenous molecules can include proteins, for example, transcription factors and enzymes.

As used herein, the term "product of an exogenous nucleic acid" includes both polynucleotide and polypeptide products, for example, transcription products (polynucleotides such as RNA) and translation products (polypeptides).

A "fusion" molecule is a molecule in which two or more subunit molecules are linked, preferably covalently. The subunit molecules can be the same chemical type of molecule, or can be different chemical types of molecules. Examples of fusion molecules include, but are not limited to, fusion proteins (for example, a fusion between a protein DNA-binding domain and a cleavage domain), fusions between a polynucleotide DNA-binding domain (*e*.*g*., sgRNA) operatively associated with a cleavage domain, and fusion nucleic acids (for example, a nucleic acid encoding the fusion protein).

Expression of a fusion protein in a cell can result from delivery of the fusion protein to the cell or by delivery of a polynucleotide encoding the fusion protein to a cell, wherein the polynucleotide is transcribed, and the transcript is translated, to generate the fusion protein. Trans-splicing, polypeptide cleavage and polypeptide ligation can also be involved in expression of a protein in a cell. Methods for polynucleotide and polypeptide delivery to cells are presented elsewhere in this disclosure.

A "gene," for the purposes of the present disclosure, includes a DNA region encoding a gene product (see *infra*), as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites and locus control regions.

"Gene expression" refers to the conversion of the information contained in a gene, into a gene product. A gene product can be the direct transcriptional product of a gene (*e.g*., mRNA, tRNA, rRNA, antisense RNA, ribozyme, structural RNA or any other type of RNA) or a protein produced by translation of an mRNA. Gene products also include RNAs which are modified, by processes such as capping, polyadenylation, methylation, and editing, and proteins modified by, for example, methylation, acetylation, phosphorylation, ubiquitination, ADP-ribosylation, myristilation, and glycosylation.

"Modulation" of gene expression refers to a change in the activity of a gene. Modulation of expression can include, but is not limited to, gene activation and gene repression. Genome editing (*e*.*g*., cleavage, alteration, inactivation, random mutation) can be used to modulate expression. Gene inactivation refers to any reduction in gene expression as compared to a cell that does not include a ZFP, TALE or CRISPR/Cas system as described herein. Thus, gene inactivation may be partial or complete.

A "region of interest" is any region of cellular chromatin, such as, for example, a gene or a non-coding sequence within or adjacent to a gene, in which it is desirable to bind an exogenous molecule. Binding can be for the purposes of targeted DNA cleavage and/or targeted recombination. A region of interest can be present in a chromosome, an episome, an organellar genome (*e*.*g*., mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region. A region of interest can be as small as a single nucleotide pair or up to 2,000 nucleotide pairs in length, or any integral value of nucleotide pairs.

A "safe harbor" locus is a locus within the genome wherein a gene may be inserted without any deleterious effects on the host cell. Most beneficial is a safe harbor locus in which expression of the inserted gene sequence is not perturbed by any read-through expression from neighboring genes. Non-limiting examples of safe harbor loci that are targeted by nuclease(s) include CCR5, HPRT, AAVS1, *Rosa* and albumin. *See, e.g.,* U.S. Patent Nos. 7,951,925; 8,771,985; 8,110,379; 7,951,925; U.S. Publication Nos. 20100218264; 20110265198; 20130137104; 20130122591; 20130177983; 20130177960; 20150056705 and 20150159172.

A "reporter gene" or "reporter sequence" refers to any sequence that produces a protein product that is easily measured, preferably although not necessarily in a routine assay. Suitable reporter genes include, but are not limited to, sequences encoding proteins that mediate antibiotic resistance (*e*.*g*., ampicillin resistance, neomycin resistance, G418 resistance, puromycin resistance), sequences encoding colored or fluorescent or luminescent proteins (*e*.*g*., green fluorescent protein, enhanced green fluorescent protein, red fluorescent protein, luciferase), and proteins which mediate enhanced cell growth and/or gene amplification (*e*.*g*., dihydrofolate reductase). Epitope tags include, for example, one or more copies of FLAG, His, myc, Tap, HA or any detectable amino acid sequence. "Expression tags" include sequences that encode reporters that may be operably linked to a desired gene sequence in order to monitor expression of the gene of interest.

"Eukaryotic" cells include, but are not limited to, fungal cells (such as yeast), plant cells, animal cells, mammalian cells and human cells (*e.g.,* T-cells), including stem cells (pluripotent and multipotent).

The terms "operative linkage" and "operatively linked" (or "operably linked") are used interchangeably with reference to a juxtaposition of two or more components (such as sequence elements), in which the components are arranged such that both components function normally and allow the possibility that at least one of the components can mediate a function that is exerted upon at least one of the other components. By way of illustration, a transcriptional regulatory sequence, such as a promoter, is operatively linked to a coding sequence if the transcriptional regulatory sequence controls the level of transcription of the coding sequence in response to the presence or absence of one or more transcriptional regulatory factors. A transcriptional regulatory sequence is generally operatively linked in *cis* with a coding sequence, but need not be directly adjacent to it. For example, an enhancer is a transcriptional regulatory sequence that is operatively linked to a coding sequence, even though they are not contiguous.

A "functional fragment" of a protein, polypeptide or nucleic acid is a protein, polypeptide or nucleic acid whose sequence is not identical to the full-length protein, polypeptide or nucleic acid, yet retains the same function as the full-length protein, polypeptide or nucleic acid. A functional fragment can possess more, fewer, or the same number of residues as the corresponding native molecule, and/or can contain one or more amino acid or nucleotide substitutions. Methods for determining the function of a nucleic acid or protein (*e*.*g*., coding function, ability to hybridize to another nucleic acid, enzymatic activity assays) are well-known in the art.

A polynucleotide "vector" or "construct" is capable of transferring gene sequences to target cells. Typically, "vector construct," "expression vector," "expression construct," "expression cassette," and "gene transfer vector," mean any nucleic acid construct capable of directing the expression of a gene of interest and which can transfer gene sequences to target cells. Thus, the term includes cloning, and expression vehicles, as well as integrating vectors.

The terms "subject" and "patient" are used interchangeably and refer to mammals such as human patients and non-human primates, as well as experimental animals such as rabbits, dogs, cats, rats, mice, and other animals. Accordingly, the term "subject" or "patient" as used herein means any mammalian patient or subject to which the expression cassettes of the invention can be administered. Subjects of the present invention include those with a disorder.

The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage. Cancer, monogenic diseases and graft versus host disease are non-limiting examples of conditions that may be treated using the compositions and methods described herein.

"Chromatin" is the nucleoprotein structure comprising the cellular genome. Cellular chromatin comprises nucleic acid, primarily DNA, and protein, including histones and non-histone chromosomal proteins. The majority of eukaryotic cellular chromatin exists in the form of nucleosomes, wherein a nucleosome core comprises approximately 150 base pairs of DNA associated with an octamer comprising two each of histones H2A, H2B, H3 and H4; and linker DNA (of variable length depending on the organism) extends between nucleosome cores. A molecule of histone H1 is generally associated with the linker DNA. For the purposes of the present disclosure, the term "chromatin" is meant to encompass all types of cellular nucleoprotein, both prokaryotic and eukaryotic. Cellular chromatin includes both chromosomal and episomal chromatin.

An "accessible region" is a site in cellular chromatin in which a target site present in the nucleic acid can be bound by an exogenous molecule which recognizes the target site. Without wishing to be bound by any particular theory, it is believed that an accessible region is one that is not packaged into a nucleosomal structure. The distinct structure of an accessible region can often be detected by its sensitivity to chemical and enzymatic probes, for example, nucleases.

A "target site" or "target sequence" is a nucleic acid sequence that defines a portion of a nucleic acid to which a binding molecule will bind, provided sufficient conditions for binding exist. For example, the sequence 5'-GAATTC-3' is a target site for the Eco RI restriction endonuclease. An "intended" or "on-target" sequence is the sequence to which the binding molecule is intended to bind and an "unintended" or "off-target" sequence includes any sequence bound by the binding molecule that is not the intended target.

### DNA-binding molecules/domains

Described herein are compositions comprising a DNA-binding molecule/domain that specifically binds to a target site in any gene or locus of interest. Any DNA-binding molecule/domain can be used in the compositions and methods disclosed herein, including but not limited to a zinc finger DNA-binding domain, a TALE DNA binding domain, the DNA-binding portion (guide or sgRNA) of a CRISPR/Cas nuclease, or a DNA-binding domain from a meganuclease.

In certain embodiments, the DNA binding domain comprises a zinc finger protein. Preferably, the zinc finger protein is non-naturally occurring in that it is engineered to bind to a target site of choice. *See,* for example, Beerli et al. (2002) Nature Biotechnol. 20:135-141; Pabo et al. (2001) Ann, Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416; U.S. Patent Nos. 6,453,242; 6,534,261; 6,599,692; 6,503,717; 6,689,558; 7,030,215; 6,794,136; 7,067,317; 7,262,054; 7,070,934; 7,361,635; 7,253,273; and U.S. Patent Publication Nos. 2005/0064474; 2007/0218528; 2005/0267061, all incorporated herein by reference in their entireties.

In certain embodiments, the DNA-binding domain comprises a zinc finger protein that binds to a target site in a PD1 gene, for example, as disclosed in U.S. Patent Publication No. 2012/0060230 (*e.g.,* Table 1), incorporated by reference in its entirety herein. Non-limiting examples of suitable ZFPs include ZFPs designated 12942 and 25029, having the recognition helix regions shown in Tables 2 and 3 of U.S. U.S. Patent No. 8,563,314. In certain embodiments, the ZFP DNA-binding domains have the amino acid sequence as shown in SEQ ID NO:3 or SEQ ID NO:5.

An engineered zinc finger binding domain can have a novel binding specificity, compared to a naturally-occurring zinc finger protein. Engineering methods include, but are not limited to, rational design and various types of selection. Rational design includes, for example, using databases comprising triplet (or quadruplet) nucleotide sequences and individual zinc finger amino acid sequences, in which each triplet or quadruplet nucleotide sequence is associated with one or more amino acid sequences of zinc fingers which bind the particular triplet or quadruplet sequence. See, for example, U.S. Patents 6,453,242 and 6,534,261, incorporated by reference herein in their entireties.

Exemplary selection methods, including phage display and two-hybrid systems, are disclosed in US Patents 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237. In addition, enhancement of binding specificity for zinc finger binding domains has been described, for example, in U.S. Patent No. 6,794,136.

In addition, as disclosed in these and other references, zinc finger domains and/or multi-fingered zinc finger proteins may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein. In addition, enhancement of binding specificity for zinc finger binding domains has been described, for example, in U.S. Patent No. 6,794,136.

Selection of target sites; ZFPs and methods for design and construction of fusion proteins (and polynucleotides encoding same) are known to those of skill in the art and described in detail in U.S. Patent Nos. 6,140,081; 5,789,538; 6,453,242; 6,534,261; 5,925,523; 6,007,988; 6,013,453; 6,200,759; WO 95/19431; WO 96/06166; WO 98/53057; WO 98/54311; WO 00/27878; WO 01/60970 WO 01/88197; WO 02/099084; WO 98/53058; WO 98/53059; WO 98/53060; WO 02/016536 and WO 03/016496.

In addition, as disclosed in these and other references, zinc finger domains and/or multi-fingered zinc finger proteins may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein.

Usually, the ZFPs include at least three fingers. Certain of the ZFPs include four, five or six or more fingers. The ZFPs that include three fingers typically recognize a target site that includes 9 or 10 nucleotides; ZFPs that include four fingers typically recognize a target site that includes 12 to 14 nucleotides; while ZFPs having six fingers can recognize target sites that include 18 to 21 nucleotides. The ZFPs can also be fusion proteins that include one or more regulatory domains, which domains can be transcriptional activation or repression domains.

In some embodiments, the DNA-binding domain may be derived from a nuclease. For example, the recognition sequences of homing endonucleases and meganucleases such as I-*Sce*I, I*-Ceu*I*,* PI*-Psp*I*,* PI-*Sce*, I*-Sce*IV*,* I-*Csm*I, I*-Pan*I, I-*Sce*II, I*-Ppo*I*,* I-*Sce*III, I*-Cre*I*,* I*-Tev*I*,* I*-Tev*II and I-*Tev*III are known. *See* also U.S. Patent No. 5,420,032; U.S. Patent No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue. In addition, the DNA-binding specificity of homing endonucleases and meganucleases can be engineered to bind non-natural target sites. *See,* for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication No. 20070117128.

In certain embodiments, the zinc finger protein used with the mutant cleavage domains described herein comprises one or more mutations (substitutions, deletions, and/or insertions) to the backbone regions (e.g., regions outside the 7-amino acid recognition helix region numbered -1 to 6), for example at one or more of positions -14, -9 and/or -5. The wild-type residue at one or more these positions may be deleted, replaced with any amino acid residue and/or include on or more additional residues. In some embodiments, the Arg (R) at position -5 is changed to a Tyr (Y), Asp (N), Glu (E), Leu (L), Gln (Q), or Ala (A). In other embodiments, the Arg (R) at position (-9) is replaced with Ser (S), Asp (N), or Glu (E). In further embodiments, the Arg (R) at position (-14) is replaced with Ser (S) or Gin (Q). In other embodiments, the fusion polypeptides can comprise mutations in the zinc finger DNA binding domain where the amino acids at the (-5), (-9) and/or (-14) positions in one or more fingers are changed to any of the above listed amino acids in any combination, for example backbone mutations (*e*.*g*., (-5) mutations) to 1, 2, 3, 4, 5, or 6 fingers of a zinc finger protein.

In other embodiments, the DNA binding domain comprises an engineered domain from a Transcriptional Activator-Like (TAL) effector (TALE) similar to those derived from the plant pathogens *Xanthomonas* (see Boch et al, (2009) Science 326: 1509-1512 and Moscou and Bogdanove, (2009) Science 326: 1501) and Ralstonia (see Heuer et al (2007) Applied and Environmental Microbiology 73(13): 4379-4384); U.S. Patent Publication Nos. 20110301073 and 20110145940. The plant pathogenic bacteria of the genus *Xanthomonas* are known to cause many diseases in important crop plants. Pathogenicity of *Xanthomonas* depends on a conserved type III secretion (T3S) system which injects more than 25 different effector proteins into the plant cell. Among these injected proteins are transcription activator-like effectors (TALE) which mimic plant transcriptional activators and manipulate the plant transcriptome (see Kay et al (2007) Science318:648-651*).* These proteins contain a DNA binding domain and a transcriptional activation domain. One of the most well characterized TALEs is AvrBs3 from *Xanthomonas campestgris* pv. *Vesicatoria* (see Bonas et al (1989) Mal Gen Genet 218: 127-136 and WO2010079430). TALEs contain a centralized domain of tandem repeats, each repeat containing approximately 34 amino acids, which are key to the DNA binding specificity of these proteins. In addition, they contain a nuclear localization sequence and an acidic transcriptional activation domain (for a review see Schornack S, et al (2006) J Plant Physiol 163(3): 256-272). In addition, in the phytopathogenic bacteria *Ralstonia solanacearum* two genes, designated brg11 and hpx17 have been found that are homologous to the AvrBs3 family *of Xanthomonas* in the *R. solanacearum* biovar 1 strain GMI1000 and in the biovar 4 strain RS1000 (*See* Heuer et al (2007) Appl and Envir Micro 73(13): 4379-4384). These genes are 98.9% identical in nucleotide sequence to each other but differ by a deletion of 1,575 base pairs in the repeat domain of hpx17. However, both gene products have less than 40% sequence identity with AvrBs3 family proteins *of Xanthomonas.*

Specificity of these TAL effectors depends on the sequences found in the tandem repeats. The repeated sequence comprises approximately 102 base pairs and the repeats are typically 91-100% homologous with each other (Bonas *et al, ibid*). Polymorphism of the repeats is usually located at positions 12 and 13 and there appears to be a one-to-one correspondence between the identity of the hypervariable diresidues (the repeat variable diresidue or RVD region) at positions 12 and 13 with the identity of the contiguous nucleotides in the TAL-effector's target sequence (see Moscou and Bogdanove, (2009) Science 326:1501 and Boch et al (2009) Science 326:1509-1512). Experimentally, the natural code for DNA recognition of these TAL-effectors has been determined such that an HD sequence at positions 12 and 13 (Repeat Variable Diresidue or RVD) leads to a binding to cytosine (C), NG binds to T, NI to A, C, G or T, NN binds to A or G, and ING binds to T. These DNA binding repeats have been assembled into proteins with new combinations and numbers of repeats, to make artificial transcription factors that are able to interact with new sequences and activate the expression of a non-endogenous reporter gene in plant cells (Boch *et al, ibid*). Engineered TAL proteins have been linked to a *Fok*I cleavage half domain to yield a TAL effector domain nuclease fusion (TALEN), including TALENs with atypical RVDs. *See, e.g.,* U.S. Patent No. 8,586,526.

In some embodiments, the TALEN comprises an endonuclease (*e*.*g*., *Fok*I) cleavage domain or cleavage half-domain. In other embodiments, the TALE-nuclease is a mega TAL. These mega TAL nucleases are fusion proteins comprising a TALE DNA binding domain and a meganuclease cleavage domain. The meganuclease cleavage domain is active as a monomer and does not require dimerization for activity. (See Boissel et al., (2013) Nucl Acid Res: 1-13, doi: 10.1093/nar/gkt1224).

In still further embodiments, the nuclease comprises a compact TALEN. These are single chain fusion proteins linking a TALE DNA binding domain to a TevI nuclease domain. The fusion protein can act as either a nickase localized by the TALE region, or can create a double strand break, depending upon where the TALE DNA binding domain is located with respect to the TevI nuclease domain (see Beurdeley et al (2013) Nat Comm: 1-8 DOI: 10.1038/ncomms2782). In addition, the nuclease domain may also exhibit DNA-binding functionality. Any TALENs may be used in combination with additional TALENs (*e*.*g*., one or more TALENs (cTALENs or *Fok*I-TALENs) with one or more mega-TALEs.

In addition, as disclosed in these and other references, zinc finger domains and/or multi-fingered zinc finger proteins or TALEs may be linked together using any suitable linker sequences, including for example, linkers of 5 or more amino acids in length. See, also, U.S. Patent Nos. 6,479,626; 6,903,185; and 7,153,949 for exemplary linker sequences 6 or more amino acids in length. The proteins described herein may include any combination of suitable linkers between the individual zinc fingers of the protein. In addition, enhancement of binding specificity for zinc finger binding domains has been described, for example, in U.S. Patent No. 6,794,136.In certain embodiments, the DNA-binding domain is part of a CRISPR/Cas nuclease system, including a single guide RNA (sgRNA) DNA binding molecule that binds to DNA. *See, e.g.,* U.S. Patent No. 8,697,359 and U.S. Patent Publication Nos. 20150056705 and 20150159172. The CRISPR (clustered regularly interspaced short palindromic repeats) locus, which encodes RNA components of the system, and the cas (CRISPR-associated) locus, which encodes proteins (Jansen et al., 2002. Mol. Microbiol. 43: 1565-1575; Makarova et al., 2002. Nucleic Acids Res. 30: 482-496; Makarova et al., 2006. Biol. Direct 1: 7; Haft et al., 2005. PLoS Comput. Biol. 1: e60) make up the gene sequences of the CRISPR/Cas nuclease system. CRISPR loci in microbial hosts contain a combination of CRISPR-associated (Cas) genes as well as non-coding RNA elements capable of programming the specificity of the CRISPR-mediated nucleic acid cleavage.

In some embodiments, the DNA binding domain is part of a TtAgo system (see Swarts *et al, ibid*; Sheng *et al, ibid*). In eukaryotes, gene silencing is mediated by the Argonaute (Ago) family of proteins. In this paradigm, Ago is bound to small (19-31 nt) RNAs. This protein-RNA silencing complex recognizes target RNAs via Watson-Crick base pairing between the small RNA and the target and endonucleolytically cleaves the target RNA (Vogel (2014) Science 344:972-973). In contrast, prokaryotic Ago proteins bind to small single-stranded DNA fragments and likely function to detect and remove foreign (often viral) DNA (Yuan et al., (2005) Mol. Cell 19, 405; Olovnikov, et al. (2013) Mol. Cell 51, 594; Swarts *et al.*, *ibid*). Exemplary prokaryotic Ago proteins include those from *Aquifex aeolicus*, *Rhodobacter sphaeraides,* and *Thermus thermophilus.*

One of the most well-characterized prokaryotic Ago protein is the one from *T. thermophilus* (TtAgo; Swarts *et al. ibid*). TtAgo associates with either 15 nt or 13-25 nt single-stranded DNA fragments with 5' phosphate groups. This "guide DNA" bound by TtAgo serves to direct the protein-DNA complex to bind a Watson-Crick complementary DNA sequence in a third-party molecule of DNA. Once the sequence information in these guide DNAs has allowed identification of the target DNA, the TtAgo-guide DNA complex cleaves the target DNA. Such a mechanism is also supported by the structure of the TtAgo-guide DNA complex while bound to its target DNA (G. Sheng *et al.*, *ibid*). Ago from *Rhodobacter sphaeroides* (RsAgo) has similar properties (Olivnikov *et al. ibid*)*.*

Exogenous guide DNAs of arbitrary DNA sequence can be loaded onto the TtAgo protein (Swarts *et al. ibid.*)*.* Since the specificity of TtAgo cleavage is directed by the guide DNA, a TtAgo-DNA complex formed with an exogenous, investigator-specified guide DNA will therefore direct TtAgo target DNA cleavage to a complementary investigator-specified target DNA. In this way, one may create a targeted double-strand break in DNA. Use of the TtAgo-guide DNA system (or orthologous Ago-guide DNA systems from other organisms) allows for targeted cleavage of genomic DNA within cells. Such cleavage can be either single- or double-stranded. For cleavage of mammalian genomic DNA, it would be preferable to use of a version of TtAgo codon optimized for expression in mammalian cells. Further, it might be preferable to treat cells with a TtAgo-DNA complex formed *in vitro* where the TtAgo protein is fused to a cell-penetrating peptide. Further, it might be preferable to use a version of the TtAgo protein that has been altered via mutagenesis to have improved activity at 37°C. Ago-RNA-mediated DNA cleavage could be used to affect a panopoly of outcomes including gene knock-out, targeted gene addition, gene correction, targeted gene deletion using techniques standard in the art for exploitation of DNA breaks.

Thus, any DNA-binding molecule/domain can be used.

### Fusion molecules

Fusion molecules comprising DNA-binding domains (e.g., ZFPs or TALEs, CRISPR/Cas components such as single guide RNAs) as described herein and a heterologous regulatory (functional) domain (or functional fragment thereof) are also provided. Common domains include, *e*.*g*., transcription factor domains (activators, repressors, co-activators, co-repressors), silencers, oncogenes (*e*.*g*., myc, jun, fos, myb, max, mad, rel, ets, bcl, myb, mos family members etc.); DNA repair enzymes and their associated factors and modifiers; DNA rearrangement enzymes and their associated factors and modifiers; chromatin associated proteins and their modifiers (*e*.*g*. kinases, acetylases and deacetylases); and DNA modifying enzymes (*e*.*g*., methyltransferases, topoisomerases, helicases, ligases, kinases, phosphatases, polymerases, endonucleases) and their associated factors and modifiers. U.S. Patent Publication Nos. 20050064474; 20060188987 and 2007/0218528 for details regarding fusions of DNA-binding domains and nuclease cleavage domains, incorporated by reference in their entireties herein.

Suitable domains for achieving activation include the HSV VP16 activation domain (see, *e*.*g*., Hagmann et al., J. Virol. 71, 5952-5962 (1997)) nuclear hormone receptors (see, *e*.*g*., Torchia et al., Curr. Opin. Cell. Biol. 10:373-383 (1998)); the p65 subunit of nuclear factor kappa B (Bitko & Barik, J. Virol. 72:5610-5618 (1998) and Doyle & Hunt, Neuroreport 8:2937-2942 (1997)); Liu et al., Cancer Gene Ther. 5:3-28 (1998)), or artificial chimeric functional domains such as VP64 (Beerli et al., (1998) Proc. Natl. Acad. Sci. USA 95:14623-33), and degron (Molinari et al., (1999) EMBO J. 18, 6439-6447). Additional exemplary activation domains include, Oct 1, Oct-2A, Spl, AP-2, and CTF1 (Seipel etal., EMBO J. 11, 4961-4968 (1992) as well as p300, CBP, PCAF, SRC1 PvALF, AtHD2A and ERF-2. See, for example, Robyr et al. (2000) Mol. Endocrinol. 14:329-347; Collingwood et al. (1999) J. Mol. Endocrinol. 23:255-275; Leo et al. (2000) Gene 245:1-11; Manteuffel-Cymborowska (1999) Acta Biochim. Pol. 46:77-89; McKenna et al. (1999) J. Steroid Biochem. Mol. Biol. 69:3-12; Malik et al. (2000) Trends Biochem. Sci. 25:277-283; and Lemon et al. (1999) Curr. Opin. Genet. Dev. 9:499-504. Additional exemplary activation domains include, but are not limited to, OsGAI, HALF-1, C1, AP1, ARF-5,-6,-7, and -8, CPRF1, CPRF4, MYC-RP/GP, and TRAB1. See, for example, Ogawa et al. (2000) Gene 245:21-29; Okanami et al. (1996) Genes Cells 1:87-99; Goff et al. (1991) Genes Dev. 5:298-309; Cho et al. (1999) Plant Mol. Biol. 40:419-429; Ulmason et al. (1999) Proc. Natl. Acad. Sci. USA 96:5844-5849; Sprenger-Haussels et al. (2000) Plant J. 22:1-8; Gong et al. (1999) Plant Mol. Biol. 41:33-44; and Hobo et al. (1999) Proc. Natl. Acad. Sci. USA 96:15,348-15,353.

It will be clear to those of skill in the art that, in the formation of a fusion protein (or a nucleic acid encoding same) between a DNA-binding domain and a functional domain, either an activation domain or a molecule that interacts with an activation domain is suitable as a functional domain. Essentially any molecule capable of recruiting an activating complex and/or activating activity (such as, for example, histone acetylation) to the target gene is useful as an activating domain of a fusion protein. Insulator domains, localization domains, and chromatin remodeling proteins such as ISWI-containing domains and/or methyl binding domain proteins suitable for use as functional domains in fusion molecules are described, for example, in U.S. Patent Publications 2002/0115215 and 2003/0082552 and in WO 02/44376.

Exemplary repression domains include, but are not limited to, KRAB A/B, KOX, TGF-beta-inducible early gene (TIEG), v-erbA, SID, MBD2, MBD3, members of the DNMT family (e.g., DNMT1, DNMT3A, DNMT3B), Rb, and MeCP2. See, for example, Bird et al. (1999) Cell 99:451-454; Tyler et al. (1999) Cell 99:443-446; Knoepfler et al. (1999) Cell 99:447-450; and Robertson et al. (2000) Nature Genet. 25:338-342. Additional exemplary repression domains include, but are not limited to, ROM2 and AtHD2A. See, for example, Chem et al. (1996) Plant Cell 8:305-321; and Wu et al. (2000) Plant J. 22:19-27.

Fusion molecules are constructed by methods of cloning and biochemical conjugation that are well known to those of skill in the art. Fusion molecules comprise a DNA-binding domain and a functional domain (*e*.*g*., a transcriptional activation or repression domain). Fusion molecules also optionally comprise nuclear localization signals (such as, for example, that from the SV40 medium T-antigen) and epitope tags (such as, for example, FLAG and hemagglutinin). Fusion proteins (and nucleic acids encoding them) are designed such that the translational reading frame is preserved among the components of the fusion.

Fusions between a polypeptide component of a functional domain (or a functional fragment thereof) on the one hand, and a non-protein DNA-binding domain (e.g., antibiotic, intercalator, minor groove binder, nucleic acid) on the other, are constructed by methods of biochemical conjugation known to those of skill in the art. See, for example, the Pierce Chemical Company (Rockford, IL) Catalogue. Methods and compositions for making fusions between a minor groove binder and a polypeptide have been described. Mapp et al. (2000) Proc. Natl. Acad. Sci. USA 97:3930-3935. Furthermore, single guide RNAs of the CRISPR/Cas system associate with functional domains to form active transcriptional regulators and nucleases.

In certain embodiments, the target site is present in an accessible region of cellular chromatin. Accessible regions can be determined as described, for example, in U.S. Patent Nos. 7,217,509 and 7,923,542. If the target site is not present in an accessible region of cellular chromatin, one or more accessible regions can be generated as described in U.S. Patent Nos. 7,785,792 and 8,071,370. In additional embodiments, the DNA-binding domain of a fusion molecule is capable of binding to cellular chromatin regardless of whether its target site is in an accessible region or not. For example, such DNA-binding domains are capable of binding to linker DNA and/or nucleosomal DNA. Examples of this type of "pioneer" DNA binding domain are found in certain steroid receptor and in hepatocyte nuclear factor 3 (HNF3) (Cordingley et al. (1987) Cell 48:261-270; Pina et al. (1990) Cell 60:719-731; and Cirillo et al. (1998) EMBO J. 17:244-254).

The fusion molecule may be formulated with a pharmaceutically acceptable carrier, as is known to those of skill in the art. See, for example, Remington's Pharmaceutical Sciences, 17th ed., 1985; and U.S. Patent Nos. 6,453,242 and 6,534,261.

The functional component/domain of a fusion molecule can be selected from any of a variety of different components capable of influencing transcription of a gene once the fusion molecule binds to a target sequence via its DNA binding domain. Hence, the functional component can include, but is not limited to, various transcription factor domains, such as activators, repressors, co-activators, co-repressors, and silencers.

Additional exemplary functional domains are disclosed, for example, in U.S. Patent Nos. 6,534,261 and 6,933,113.

Functional domains that are regulated by exogenous small molecules or ligands may also be selected. For example, RheoSwitch^{®} technology may be employed wherein a functional domain only assumes its active conformation in the presence of the external RheoChem^{™} ligand (see for example US 20090136465). Thus, the ZFP may be operably linked to the regulatable functional domain wherein the resultant activity of the ZFP-TF is controlled by the external ligand.

### Nucleases

In certain embodiments, the fusion protein comprises a DNA-binding binding domain and cleavage (nuclease) domain. As such, gene modification can be achieved using a nuclease, for example an engineered nuclease. Engineered nuclease technology is based on the engineering of naturally occurring DNA-binding proteins. For example, engineering of homing endonucleases with tailored DNA-binding specificities has been described. Chames et al. (2005) Nucleic Acids Res 33(20):e178; Amould et al. (2006) J. Mol. Biol. 355:443-458. In addition, engineering of ZFPs has also been described. See, e.g., U.S. Patent Nos. 6,534,261; 6,607,882; 6,824,978; 6,979,539; 6,933,113; 7,163,824; and 7,013,219.

In addition, ZFPs and/or TALEs have been fused to nuclease domains to create ZFNs and TALENs - a functional entity that is able to recognize its intended nucleic acid target through its engineered (ZFP or TALE) DNA binding domain and cause the DNA to be cut near the DNA binding site via the nuclease activity. See, *e.g.,* Kim et al. (1996) Proc Nat'l Acad Sci USA 93(3):1156-1160. More recently, such nucleases have been used for genome modification in a variety of organisms. See, for example, United States Patent Publications 20030232410; 20050208489; 20050026157; 20050064474; 20060188987; 20060063231; and International Publication WO 07/014275.

Thus, the methods and compositions described herein are broadly applicable and may involve any nuclease of interest. Non-limiting examples of nucleases include meganucleases, TALENs and zinc finger nucleases. The nuclease may comprise heterologous DNA-binding and cleavage domains (e.g., zinc finger nucleases; meganuclease DNA-binding domains with heterologous cleavage domains) or, alternatively, the DNA-binding domain of a naturally-occurring nuclease may be altered to bind to a selected target site (e.g., a meganuclease that has been engineered to bind to site different than the cognate binding site).

In any of the nucleases described herein, the nuclease can comprise an engineered TALE DNA-binding domain and a nuclease domain (*e*.*g*., endonuclease and/or meganuclease domain), also referred to as TALENs. Methods and compositions for engineering these TALEN proteins for robust, site specific interaction with the target sequence of the user's choosing have been published (see U.S. Patent No. 8,586,526). In some embodiments, the TALEN comprises an endonuclease (*e.g., Fok*I) cleavage domain or cleavage half-domain. In other embodiments, the TALE-nuclease is a mega TAL. These mega TAL nucleases are fusion proteins comprising a TALE DNA binding domain and a meganuclease cleavage domain. The meganuclease cleavage domain is active as a monomer and does not require dimerization for activity. (See Boissel et al., (2013) Nucl Acid Res: 1-13, doi: 10.I0931nar/gkt1224). In addition, the nuclease domain may also exhibit DNA-binding functionality.

In still further embodiments, the nuclease comprises a compact TALEN (cTALEN). These are single chain fusion proteins linking a TALE DNA binding domain to a TevI nuclease domain. The fusion protein can act as either a nickase localized by the TALE region, or can create a double strand break, depending upon where the TALE DNA binding domain is located with respect to the *Tev*I nuclease domain (see Beurdeley et al (2013) Nat Comm: 1-8 DOI: 10.10381ncomms2782). Any TALENs may be used in combination with additional TALENs (*e*.*g*., one or more TALENs (cTALENs or FokI-TALENs) with one or more mega-TALs) or other DNA cleavage enzymes.

In certain embodiments, the nuclease comprises a meganuclease (homing endonuclease) or a portion thereof that exhibits cleavage activity. Naturally-occurring meganucleases recognize 15-40 base-pair cleavage sites and are commonly grouped into four families: the LAGLIDADG family ("LAGLIDADG" disclosed as SEQ ID NO: 6), the GIY-YIG family, the His-Cyst box family and the HNH family. Exemplary homing endonucleases include I-SceI, I-CeuI, PI-PspI, PI-Sce, I-SceIV, I-CsmI, I-PanI, I-SceII, I-PpoI, I-SceIII, I-CreI, I-TevI, I-TevII and I-TevIII. Their recognition sequences are known. See also U.S. Patent No. 5,420,032; U.S. Patent No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue.

DNA-binding domains from naturally-occurring meganucleases, primarily from the LAGLIDADG family ("LAGLIDADG" disclosed as SEQ ID NO: 6), have been used to promote site-specific genome modification in plants, yeast, Drosophila, mammalian cells and mice, but this approach has been limited to the modification of either homologous genes that conserve the meganuclease recognition sequence (Monet et al. (1999), Biochem. Biophysics. Res. Common. 255: 88-93) or to pre-engineered genomes into which a recognition sequence has been introduced (Route et al. (1994), Mol. Cell. Biol. 14: 8096-106; Chilton et al. (2003), Plant Physiology. 133: 956-65; Puchta et al. (1996), Proc. Natl. Acad. Sci. USA 93: 5055-60; Rong et al. (2002), Genes Dev. 16: 1568-81; Gouble et al. (2006), J. Gene Med. 8(5):616-622). Accordingly, attempts have been made to engineer meganucleases to exhibit novel binding specificity at medically or biotechnologically relevant sites (Porteus et al. (2005), Nat. Biotechnol. 23: 967-73; Sussman et al. (2004), J. Mol. Biol. 342: 31-41; Epinat et al. (2003), Nucleic Acids Res. 31: 2952-62; Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66; U.S. Patent Publication Nos. 20070117128; 20060206949; 20060153826; 20060078552; and 20040002092). In addition, naturally-occurring or engineered DNA-binding domains from meganucleases can be operably linked with a cleavage domain from a heterologous nuclease (*e.g., Fok*I) and/or cleavage domains from meganucleases can be operably linked with a heterologous DNA-binding domain (*e*.*g*., ZFP or TALE).

In other embodiments, the nuclease is a zinc finger nuclease (ZFN) or TALE DNA binding domain-nuclease fusion (TALEN). ZFNs and TALENs comprise a DNA binding domain (zinc finger protein or TALE DNA binding domain) that has been engineered to bind to a target site in a gene of choice and cleavage domain or a cleavage half-domain (*e*.*g*., from a restriction and/or meganuclease as described herein).

As described in detail above, zinc finger binding domains and TALE DNA binding domains can be engineered to bind to a sequence of choice. See, for example, Beerli et al. (2002) Nature Biotechnol. 20:135-141; Pabo et al. (2001) Ann. Rev. Biochem. 70:313-340; Isalan et al. (2001) Nature Biotechnol. 19:656-660; Segal et al. (2001) Curr. Opin. Biotechnol. 12:632-637; Choo et al. (2000) Curr. Opin. Struct. Biol. 10:411-416. An engineered zinc finger binding domain or TALE protein can have a novel binding specificity, compared to a naturally-occurring protein. Engineering methods include, but are not limited to, rational design and various types of selection. Rational design includes, for example, using databases comprising triplet (or quadruplet) nucleotide sequences and individual zinc finger or TALE amino acid sequences, in which each triplet or quadruplet nucleotide sequence is associated with one or more amino acid sequences of zinc fingers or TALE repeat units which bind the particular triplet or quadruplet sequence. See, for example, U.S. Patents 6,453,242 and 6,534,261, incorporated by reference herein in their entireties.

Selection of target sites; and methods for design and construction of fusion proteins (and polynucleotides encoding same) are known to those of skill in the art and described in detail in U.S. Patent Nos. 7,888,121 and 8,409,861, incorporated by reference in their entireties herein.

In addition, as disclosed in these and other references, zinc finger domains, TALEs and/or multi-fingered zinc finger proteins may be linked together using any suitable linker sequences, including for example, linkers of 5 or more amino acids in length. See, *e.g.,* U.S. Patent Nos. 6,479,626; 6,903,185; and 7,153,949 for exemplary linker sequences 6 or more amino acids in length. The proteins described herein may include any combination of suitable linkers between the individual zinc fingers of the protein. See, also, U.S. Patent No. 8,772,453.

Thus, nucleases such as ZFNs, TALENs and/or meganucleases can comprise any DNA-binding domain and any nuclease (cleavage) domain (cleavage domain, cleavage half-domain). As noted above, the cleavage domain may be heterologous to the DNA-binding domain, for example a zinc finger or TAL-effector DNA-binding domain and a cleavage domain from a nuclease or a meganuclease DNA-binding domain and cleavage domain from a different nuclease. Heterologous cleavage domains can be obtained from any endonuclease or exonuclease. Exemplary endonucleases from which a cleavage domain can be derived include, but are not limited to, restriction endonucleases and homing endonucleases. See, for example, 2002-2003 Catalogue, New England Biolabs, Beverly, MA; and Belfort et al. (1997) Nucleic Acids Res. 25:3379-3388. Additional enzymes which cleave DNA are known (*e.g.,* S1 Nuclease; mung bean nuclease; pancreatic DNase I; micrococcal nuclease; yeast HO endonuclease; see also Linn et al. (eds.) Nucleases, Cold Spring Harbor Laboratory Press,1993). One or more of these enzymes (or functional fragments thereof) can be used as a source of cleavage domains and cleavage half-domains.

Similarly, a cleavage half-domain can be derived from any nuclease or portion thereof, as set forth above, that requires dimerization for cleavage activity. In general, two fusion proteins are required for cleavage if the fusion proteins comprise cleavage half-domains. Alternatively, a single protein comprising two cleavage half-domains can be used. The two cleavage half-domains can be derived from the same endonuclease (or functional fragments thereof), or each cleavage half-domain can be derived from a different endonuclease (or functional fragments thereof). In addition, the target sites for the two fusion proteins are preferably disposed, with respect to each other, such that binding of the two fusion proteins to their respective target sites places the cleavage half-domains in a spatial orientation to each other that allows the cleavage half-domains to form a functional cleavage domain, *e*.*g*., by dimerizing. Thus, in certain embodiments, the near edges of the target sites are separated by 5-10 nucleotides or by 15-18 nucleotides. However, any integral number of nucleotides or nucleotide pairs can intervene between two target sites (e.g., from 2 to 50 nucleotide pairs or more). In general, the site of cleavage lies between the target sites.

Restriction endonucleases (restriction enzymes) are present in many species and are capable of sequence-specific binding to DNA (at a recognition site), and cleaving DNA at or near the site of binding. Certain restriction enzymes (*e*.*g*., Type IIS) cleave DNA at sites removed from the recognition site and have separable binding and cleavage domains. For example, the Type IIS enzyme *Fok*I catalyzes double-stranded cleavage of DNA, at 9 nucleotides from its recognition site on one strand and 13 nucleotides from its recognition site on the other. See, for example, US Patents 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31,978-31,982. Thus, in one embodiment, fusion proteins comprise the cleavage domain (or cleavage half-domain) from at least one Type IIS restriction enzyme and one or more zinc finger binding domains, which may or may not be engineered.

An exemplary Type IIS restriction enzyme, whose cleavage domain is separable from the binding domain, is *Fok*I*.* This particular enzyme is active as a dimer. Bitinaite et al. (1998) Proc. Natl. Acad. Sci. USA 95: 10,570-10,575. Accordingly, for the purposes of the present disclosure, the portion of the *Fok*I enzyme used in the disclosed fusion proteins is considered a cleavage half-domain. Thus, for targeted double-stranded cleavage and/or targeted replacement of cellular sequences using zinc finger-*Fok*I fusions, two fusion proteins, each comprising a *Fok*I cleavage half-domain, can be used to reconstitute a catalytically active cleavage domain. Alternatively, a single polypeptide molecule containing a zinc finger binding domain and two *Fok* I cleavage half-domains can also be used. Parameters for targeted cleavage and targeted sequence alteration using zinc finger-*Fok*I fusions are provided elsewhere in this disclosure.

A cleavage domain or cleavage half-domain can be any portion of a protein that retains cleavage activity, or that retains the ability to multimerize (*e*.*g*., dimerize) to form a functional cleavage domain.

Exemplary Type IIS restriction enzymes are described in International Publication WO 07/014275, incorporated herein in its entirety. Additional restriction enzymes also contain separable binding and cleavage domains, and these are contemplated by the present disclosure. See, for example, Roberts et al. (2003) Nucleic Acids Res. 31:418-420.

In certain embodiments, the cleavage domain comprises a cleavage domain from a *Fok*I endonuclease. The full-length *Fok*I is shown below. The cleavage domain used in the nucleases described herein is shown in italics and underlining (positions 384 to 579 of the full- length protein) where the holo protein sequence is described below (SEQ ID NO:1):

MVSKIRTFGWVQNPGKFENLKRVVQVFDRNSKVHNEVKNIKIPTLVKESKIQ
KELVAIMNQHDLIYTYKELVGTGTSIRSEAPCDAIIQATIADQGNKKGYIDNW
SSDGFLRWAHALGFIEYINKSDSFVITDVGLAYSKSADGSAIEKEILIEAISSYPP
AIRILTLLEDGQHLTKFDLGKNLGFSGESGFTSLPEGILLDTLANAMPKDKGEI
RNNWEGSSDKYARMIGGWLDKLGLVKQGKKEFIIPTLGKPDNKEFISHAFKIT
GEGLKVLRRAKGSTKFTRVPKRVYWEMLATNLTDKEYVRTRRALILEILIKA
GSLKIEQIQDNLKKLGFDEVIETIENDIKGLINTGIFIEIKGRFYQLKDHILQFVIP
NRGVTKQLVKSELEEKKSELRHKLKYVPHEYIELIEIARNSTQDRILEMKVMEFFM
KVYGYRGKHLGGSRKPDGAIYTVGSPIDYGVIVDTKAYSGGYNLPIGQADEMQRYV
EENQTRNKHINPNEWWKVYPSSVTEFKFLFVSGHFKGNYKAQLTRLNHITNCNGA
VLSVEELLIGGEMIKAGTLTLEEVRRKFNNGEINF

Cleavage half domains derived from *Fok*I may comprise a mutation in one or more of amino acid residues. Mutations include substitutions (of a wild-type amino acid residue for a different residue, insertions (of one or more amino acid residues) and/or deletions (of one or more amino acid residues). In certain embodiments, one or more of residues 414-426, 443-450, 467-488, 501-502, and/or 521-531 (numbered relative to full length sequence above) are mutated since these residues are located close to the DNA backbone in a molecular model of a ZFN bound to its target site described in Miller et al. ((2007) Nat Biotechnol 25:778-784). In certain embodiments, one or more residues at positions 416, 422, 447, 448, and/or 525 are mutated. In certain embodiments, the mutation comprises a substitution of a wild-type residue with any different residue, for example an alanine (A) residue, a cysteine (C) residue, an aspartic acid (D) residue, a glutamic acid (E) residue, a histidine (H) residue, a phenylalanine (F) residue, a glycine (G) residue, an asparagine (N) residue, a serine (S) residue or a threonine (T) residue. In other embodiments, the wild-type residue at one or more of positions 416, 418, 422, 446, 448, 476, 479, 480, 481, 525, 527 and/or 531 are replaced with any other residues, including but not limited to, R416E, R416F, R416N, S418D, S418E, R422H, N476D, N476E, N476G, N476T, I479T, I479Q, Q481A, Q481D, Q481E, Q481H, K525A, K525S, K525T, K525V, N527D, and/or Q531R.

In certain embodiments, the cleavage domain comprises one or more engineered cleavage half-domain (also referred to as dimerization domain mutants) that minimize or prevent homodimerization, as described, for example, in U.S. Patent Nos. 7,914,796; 8,034,598 and 8,623,618; and U.S. Patent Publication No. 20110201055, the disclosures of all of which are incorporated by reference in their entireties herein. Amino acid residues at positions 446, 447, 479, 483, 484,486, 487, 490, 491, 496, 498, 499, 500, 531, 534, 537, and 538 of *Fok*I (numbered relative full length *Fok*I sequence) are all targets for influencing dimerization of the *FokI* cleavage half-domains. The mutations may include mutations to residues found in natural restriction enzymes homologous to *Fok*I*.* In a preferred embodiment, the mutation at positions 416, 422, 447, 448 and/or 525 comprise replacement of a positively charged amino acid with an uncharged or a negatively charged amino acid. In another embodiment, the engineered cleavage half domain comprises mutations in amino acid residues 499, 496 and 486 in addition to the mutations in one or more amino acid residues 416, 422, 447, 448, or 525.

In certain embodiments, the compositions described herein include engineered cleavage half-domains of *Fok*I that form obligate heterodimers as described, for example, in U.S. Patent Nos. 7,914,796; 8,034,598; 8,961,281 and 8,623,618; U.S. Patent Publication Nos. 20080131962 and 20120040398. Thus, in one preferred embodiment, the invention provides fusion proteins wherein the engineered cleavage half-domain comprises a polypeptide in which the wild-type Gln (Q) residue at position 486 is replaced with a Glu (E) residue, the wild-type Ile (I) residue at position 499 is replaced with a Leu (L) residue and the wild-type Asn (N) residue at position 496 is replaced with an Asp (D) or a Glu (E) residue ("ELD" or "ELE") in addition to one or more mutations at positions 416, 422, 447, 448, or 525 (numbered relative to wild-type *Fok*I shown herein). In another embodiment, the engineered cleavage half domains are derived from a wild-type *Fok*I cleavage half domain and comprise mutations in the amino acid residues 490, 538 and 537, numbered relative to wild-type *Fok*I in addition to the one or more mutations at amino acid residues 416, 422, 447, 448, or 525. In a preferred embodiment, the invention provides a fusion protein, wherein the engineered cleavage half-domain comprises a polypeptide in which the wild-type Glu (E) residue at position 490 is replaced with a Lys (K) residue, the wild-type Ile (I) residue at position 538 is replaced with a Lys (K) residue, and the wild-type His (H) residue at position 537 is replaced with a Lys (K) residue or an Arg (R) residue ("KKK" or "KKR") (see U.S. 8,962,281, incorporated by reference herein) in addition to one or more mutations at positions 416, 422, 447, 448, or 525. See, *e.g.,* U.S. Patent Nos. 7,914,796; 8,034,598 and 8,623,618, the disclosures of which are incorporated by reference in its entirety for all purposes. In other embodiments, the engineered cleavage half domain comprises the "Sharkey" and/or "Sharkey'" mutations (see Guo et al, (2010) J. Mol. Biol. 400(1):96-107).

In another embodiment, the engineered cleavage half domains are derived from a wild-type *Fok*I cleavage half domain and comprise mutations in the amino acid residues 490, and 538, numbered relative to wild-type *Fok*I or a *Fok*I homologue in addition to the one or more mutations at amino acid residues 416, 422, 447, 448, or 525. In a preferred embodiment, the invention provides a fusion protein, wherein the engineered cleavage half-domain comprises a polypeptide in which the wild-type Glu (E) residue at position 490 is replaced with a Lys (K) residue, and the wild-type Ile (I) residue at position 538 is replaced with a Lys (K) residue ("KK") in addition to one or more mutations at positions 416, 422, 447, 448, or 525. In a preferred embodiment, the invention provides a fusion protein, wherein the engineered cleavage half-domain comprises a polypeptide in which the wild-type Gln (Q) residue at position 486 is replaced with an Glu (E) residue, and the wild-type Ile (I) residue at position 499 is replaced with a Leu (L) residue ("EL") (See U.S. 8,034,598, incorporated by reference herein) in addition to one or more mutations at positions 416, 422, 447,448, or 525.

In one aspect, the invention provides a fusion protein wherein the engineered cleavage half-domain comprises a polypeptide in which the wild-type amino acid residue at one or more of positions 387, 393, 394, 398, 400, 402, 416, 422, 427, 434, 439, 441, 447, 448, 469, 487, 495, 497, 506, 516, 525, 529, 534, 559, 569, 570, 571 in the *Fok*I catalytic domain are mutated. Nuclease domains comprising one or more mutations as shown in any of the appended Tables and Figures are provided. In some embodiments, the one or more mutations alter the wild type amino acid from a positively charged residue to a neutral residue or a negatively charged residue. In any of these embodiments, the mutants described may also be made in a *Fok*I domain comprising one or more additional mutations. In preferred embodiments, these additional mutations are in the dimerization domain, e.g. at positions 418, 432, 441, 481, 483, 486, 487, 490, 496, 499, 523, 527, 537, 538 and/or 559. Non-limiting examples of mutations include mutations (e.g., substitutions) of the wild-type residues of any cleavage domain (*e.g., Fok*I or homologue of *Fok*I) at positions 393, 394, 398, 416, 421, 422, 442, 444, 472, 473, 478, 480, 525 or 530 with any amino acid residue (e.g., K393X, K394X, R398X, R416S, D421X, R422X, K444X, S472X, G473X, S472, P478X, G480X, K525X, and A530X, where the first residue depicts wild-type and X refers to any amino acid that is substituted for the wild-type residue). In some embodiments, X is E, D, H, A, K, S, T, D or N. Other exemplary mutations include S418E, S418D, S446D, S446R, K448A, I479Q, I479T, Q481A, Q481N, Q481E, A530E and/or A530K wherein the amino acid residues are numbered relative to full length *Fok*I wild-type cleavage domain and homologues thereof. In certain embodiments, combinations may include 416 and 422, a mutation at position 416 and K448A, K448A and I479Q, K448A and Q481A and/or K448A and a mutation at position 525. In one embodiment, the wild-residue at position 416 may be replaced with a Glu (E) residue (R416E), the wild-type residue at position 422 is replaced with a His (H) residue (R422H), and the wild-type residue at position 525 is replaced with an Ala (A) residue. The cleavage domains as described herein can further include additional mutations, including but not limited to at positions 432, 441, 483, 486, 487, 490, 496, 499, 527, 537, 538 and/or 559, for example dimerization domain mutants (*e.g.,* ELD, KKR) and or nickase mutants (mutations to the catalytic domain). The cleavage half-domains with the mutations described herein form heterodimers as known in the art.

Thus, provided herein are nucleases that cleave a PD1 gene. In certain embodiments, the nuclease is a ZFN comprising a ZFN nuclease pair of left and right ZFNs, the left and right ZFNs each comprising a cleavage domain (*e.g.,* a *Fok*I cleavage domain) and a PD1-binding ZFP, wherein the cleavage domains of the left and right ZFNs dimerize and the PD1 gene is cleaved. In certain aspects, the PD1-binding ZFPs comprise ZFPs designated 12942 and 25029, the full amino acid sequences of which are shown below (SEQ ID NO:3 and SEQ ID NO:5). In other embodiments, the nuclease is a TALEN comprising a TALEN nuclease pair of left and right TALENs, the left and right TALENs each comprising a cleavage domain (*e.g.,* a *Fok*I cleavage domain) and a PD1-binding TAL-effector domain, wherein the cleavage domains of the left and right TALENs dimerize and the PD1 gene is cleaved.

One or both of the PD1 gene binding ZFNs of the pair further include one or more mutations in the *Fok*I cleavage domain at least one or more of 416, 418, 422, 476, 479, 481, 525 and/or 531, preferably at 416, 422, 476, 481, and/or 525 and even more preferably at 416, 481 and/or 525, numbered relative to the wild-type *FokI* (SEQ ID NO:1). The nuclease (cleavage) domains of one or both components of a nuclease pair may also comprise one or more mutations at positions 418, 432, 441, 448, 476, 481, 483, 486, 487, 490, 496, 499, 523, 527, 537, 538 and/or 559, including but not limited to ELD, KKR, ELE, KKS. *See, e.g.,* U.S. Patent No. 8,623,618. In certain embodiments, one or both of the PD1 nuclease pair includes a single mutation, for example at position 416 (*e.g.,* in which the wild-type R residue is substituted with an E, F, or N residue), at position 418 (*e.g.,* in which the wild- type S residue is substitute with a D or E residue), at position 422 (*e*.*g*., in which the wild-type R residue is substituted with an H residue), at position 476 (*e*.*g*., in which the wild-type N residue is substituted with a D, E, G or T residue), at position 481 (*e.g.,* in which the wild-type Q residue is substituted with a D, E or H residue), at position 525 (*e.g.,* in which the wild-type K residue is substituted with an A, S, T or V residue), at position 527 (*e.g.,* in which the wild-type N residue is substituted with a D residue), or at position 531 (*e.g.,* in which the wild-type Q residue is substituted with an R residue). *See, e.g.,* Figures 1-3. In certain embodiments, the mutation comprises a single mutation selected from the group consisting of: R416E, R416F, R416N, S418D, S418E, R422H, N476D, N476E, N476G, N476T, I479T, I479Q, Q481A, Q481D, Q481E, Q481H, K525A, K525S, K525T, K525V, N527D, Q531R mutations. Non-limiting examples of substitution mutations are shown in Figures 1-3. In still further embodiments, one or more both nucleases of a nuclease pair (ZFN or TALEN) comprises a single mutation for example at position 416 (*e.g.,* in which the wild-type R residue is substituted with an E, F, or N residue), at position 418 (*e.g.,* in which the wild- type S residue is substitute with a D or E residue), at position 422 (*e.g.,* in which the wild-type R residue is substituted with an H residue), at position 476 (*e.g.,* in which the wild-type N residue is substituted with a D, E, G or T residue), at position 481 (*e.g.,* in which the wild-type Q residue is substituted with a D, E or H residue), at position 525 (*e.g.,* in which the wild-type K residue is substituted with an A, S, T or V residue), at position 527 (*e.g.,* in which the wild-type N residue is substituted with a D residue), or at position 531 (*e.g.,* in which the wild-type Q residue is substituted with an R residue) along with additional mutations outside the single mutation, for example ELE, ELD, KKR, etc. mutation(s). Thus, one or both members of the nuclease pair includes one or more mutations as described herein.

Alternatively, nucleases may be assembled *in vivo* at the nucleic acid target site using so-called "split-enzyme" technology (see *e.g.* U.S. Patent Publication No. 20090068164). Components of such split enzymes may be expressed either on separate expression constructs, or can be linked in one open reading frame where the individual components are separated, for example, by a self-cleaving 2A peptide or IRES sequence. Components may be individual zinc finger binding domains or domains of a meganuclease nucleic acid binding domain.

Nucleases (e.g., ZFNs and/or TALENs) can be screened for activity prior to use, for example in a yeast-based chromosomal system as described in as described in U.S. Patent Nos. 9,506,120 and 8,563,314.

In certain embodiments, the nuclease comprises a CRISPR/Cas system. The CRISPR (clustered regularly interspaced short palindromic repeats) locus, which encodes RNA components of the system, and the Cas (CRISPR-associated) locus, which encodes proteins (Jansen et al., 2002. Mol. Microbiol. 43: 1565-1575; Makarova et al., 2002. Nucleic Acids Res. 30: 482-496; Makarova et al., 2006. Biol. Direct 1: 7; Haft et al., 2005. PLoS Comput. Biol. 1: e60) make up the gene sequences of the CRISPR/Cas nuclease system. CRISPR loci in microbial hosts contain a combination of CRISPR-associated (Cas) genes as well as non-coding RNA elements capable of programming the specificity of the CRISPR-mediated nucleic acid cleavage.

The Type II CRISPR is one of the most well characterized systems and carries out targeted DNA double-strand break in four sequential steps. First, two non-coding RNA, the pre-crRNA array and tracrRNA, are transcribed from the CRISPR locus. Second, tracrRNA hybridizes to the repeat regions of the pre-crRNA and mediates the processing of pre-crRNA into mature crRNAs containing individual spacer sequences. Third, the mature crRNA:tracrRNA complex directs Cas9 to the target DNA via Watson-Crick base-pairing between the spacer on the crRNA and the proto spacer on the target DNA next to the proto spacer adjacent motif (PAM), an additional requirement for target recognition. Finally, Cas9 mediates cleavage of target DNA to create a double-stranded break within the protospacer. Activity of the CRISPR/Cas system comprises of three steps: (i) insertion of alien DNA sequences into the CRISPR array to prevent future attacks, in a process called 'adaptation', (ii) expression of the relevant proteins, as well as expression and processing of the array, followed by (iii) RNA-mediated interference with the alien nucleic acid. Thus, in the bacterial cell, several of the so-called 'Cas' proteins are involved with the natural function of the CRISPR/Cas system and serve roles in functions such as insertion of the alien DNA etc.

In some embodiments, the CRISPR-Cpfl system is used. The CRISPR-Cpfl system, identified in Francisella spp, is a class 2 CRISPR-Cas system that mediates robust DNA interference in human cells. Although functionally conserved, Cpfl and Cas9 differ in many aspects including in their guide RNAs and substrate specificity (see Fagerlund et al, (2015) Genom Bio 16:251). A major difference between Cas9 and Cpfl proteins is that Cpfl does not utilize tracrRNA, and thus requires only a crRNA. The FnCpf1 crRNAs are 42-44 nucleotides long (19-nucleotide repeat and 23-25-nucleotide spacer) and contain a single stem-loop, which tolerates sequence changes that retain secondary structure. In addition, the Cpfl crRNAs are significantly shorter than the ~100-nucleotide engineered sgRNAs required by Cas9, and the PAM requirements for FnCpfl are 5'-TTN-3' and 5'-CTA-3' on the displaced strand. Although both Cas9 and Cpfl make double strand breaks in the target DNA, Cas9 uses its RuvC- and HNH-like domains to make blunt-ended cuts within the seed sequence of the guide RNA, whereas Cpfl uses a RuvC-like domain to produce staggered cuts outside of the seed. Because Cpfl makes staggered cuts away from the critical seed region, NHEJ will not disrupt the target site, therefore ensuring that Cpfl can continue to cut the same site until the desired HDR recombination event has taken place. Thus, in the methods and compositions described herein, it is understood that the term '"Cas" includes both Cas9 and Cfp1 proteins. Thus, as used herein, a "CRISPR/Cas system" refers both CRISPR/Cas and/or CRISPR/Cfp1 systems, including both nuclease and/or transcription factor systems.

In certain embodiments, Cas protein may be a "functional derivative" of a naturally occurring Cas protein. A "functional derivative" of a native sequence polypeptide is a compound having a qualitative biological property in common with a native sequence polypeptide. "Functional derivatives" include, but are not limited to, fragments of a native sequence and derivatives of a native sequence polypeptide and its fragments, provided that they have a biological activity in common with a corresponding native sequence polypeptide. A biological activity contemplated herein is the ability of the functional derivative to hydrolyze a DNA substrate into fragments. The term "derivative" encompasses both amino acid sequence variants of polypeptide, covalent modifications, and fusions thereof such as derivative Cas proteins. Suitable derivatives of a Cas polypeptide or a fragment thereof include but are not limited to mutants, fusions, covalent modifications of Cas protein or a fragment thereof. Cas protein, which includes Cas protein or a fragment thereof, as well as derivatives of Cas protein or a fragment thereof, may be obtainable from a cell or synthesized chemically or by a combination of these two procedures. The cell may be a cell that naturally produces Cas protein, or a cell that naturally produces Cas protein and is genetically engineered to produce the endogenous Cas protein at a higher expression level or to produce a Cas protein from an exogenously introduced nucleic acid, which nucleic acid encodes a Cas that is same or different from the endogenous Cas. In some case, the cell does not naturally produce Cas protein and is genetically engineered to produce a Cas protein. In some embodiments, the Cas protein is a small Cas9 ortholog for delivery via an AAV vector (Ran et al (2015) Nature 510, p. 186).

The nuclease(s) may make one or more double-stranded and/or single-stranded cuts in the target site. In certain embodiments, the nuclease comprises a catalytically inactive cleavage domain (*e.g., Fok*I and/or Cas protein). *See, e.g.,* U.S. Patent No. 9,200,266; 8,703,489 and Guillinger et al. (2014) Nature Biotech. 32(6):577-582. The catalytically inactive cleavage domain may, in combination with a catalytically active domain act as a nickase to make a single-stranded cut. Therefore, two nickases can be used in combination to make a double-stranded cut in a specific region. Additional nickases are also known in the art, for example, McCaffery et al. (2016) Nucleic Acids Res. 44(2):e11. doi: 10.1093/nar/gkv878. Epub 2015 Oct 19.

Also provided herein are cells comprising one or more ZFNs, TALENs and/or polynucleotides as described herein, as well as genetically modified cells produced from cells comprising the nucleases of the invention. Thus, the invention provides an isolated population of genetically modified cells (e.g., T cells) produced using the nuclease(s) as described herein, in which population of cells the PD1 gene is genetically modified (e.g., mutated by insertions and/or deletions (indels)) by the nuclease(s) with increased specificity as compared to PD1 nucleases not comprising the *FokI* mutation(s) described herein. Specificity can be determined in a variety of ways, including but not limited to: comparing on-target (PD1) genetic modifications and off-target (non-PD1) genetic modifications made by the nucleases; determining the fold (or percentage) difference as between on and off target genetic modifications and/or; determining the actual percentage of off-target modifications (optionally compared to on-target modifications). *See, also,* appended Figures.

In certain aspects, the isolated population of genetically modified cells produced using the nucleases has a relative on/off (PD1/non-PD1) ratio greater than the on/off ratio of genetic modifications using PD1 nucleases without *FokI* mutations as described herein. *See, e.g.,* Figure 2A and 2B. In certain embodiments, the on/off ratio of genetic modifications in the cells made by the nucleases is greater than 100, preferably greater than 150 and even more preferably, greater than 200. Thus, genetic modifications in outside of the PD1 gene (off-target mutations) made by the nucleases in these cells can be reduced by 1-100 or more-fold, including but not limited to 1-50-fold (or any value therebetween) as compared to PD1 nucleases without *Fok*I mutation(s) described herein. In certain embodiments, less than 1% *(e.g.,* less than 0.5%) of the genetic modifications made by the nuclease(s) in the isolated population of cells are outside of the PD1 gene. In still further aspects, at least 40% *(e.g.,* at least 40%, at least 45%, at least 50%, at least 60%, at least 65%, at least 70%, at least 75%, at least 80%, at least 85% or at least 90%) of the cells of the population of cells produced by the nuclease(s) as described herein include modifications (indels) to PD1 and less than 0.05% of the cells include off-target (non-PD1) genetic modifications made by the nucleases. In still further aspects, the isolated population of genetically modified cells produced using the nucleases described herein have a relative on/off (PD1/non-PD1) ratio of 150 or greater. Thus, provided herein are genetically modified cells in which the PD1 is specifically altered. The genetically modified cells described herein may include further modifications, including, for example, genetic modifications (insertions and/or deletions) made to one or more additional genes (TCR, B2M, CTLA-4, safe harbor genes). Partially or fully differentiated cells descended from the isolated population of PD1 genetically modified cells are also provided. In certain embodiments, the genetically modified cells are T cells further comprising a CAR transgene (integrated into any gene randomly or via nuclease-mediated integration) and/or one or more inactivated genes (e.g., TCR, B2M, CTLA-4).

### Delivery

The proteins (e.g., nucleases), polynucleotides and/or compositions comprising the proteins and/or polynucleotides described herein may be delivered to a target cell by any suitable means, including, for example, by injection of the protein and/or mRNA components.

Suitable cells include but not limited to eukaryotic and prokaryotic cells and/or cell lines. Non-limiting examples of such cells or cell lines generated from such cells include T-cells, COS, CHO (e.g., CHO-S, CHO-K1, CHO-DG44, CHO-DUXB11, CHO-DUKX, CHOK1SV), VERO, MDCK, WI38, V79, B14AF28-G3, BHK, HaK, NS0, SP2/0-Ag14, HeLa, HEK293 (e.g., HEK293-F, HEK293-H, HEK293-T), and perC6 cells as well as insect cells such as *Spodoptera fugiperda* (Sf), or fungal cells such as Saccharomyces, Pichia and Schizosaccharomyces. In certain embodiments, the cell line is a CHO-K1, MDCK or HEK293 cell line. Suitable cells also include stem cells such as, by way of example, embryonic stem cells, induced pluripotent stem cells (iPS cells), hematopoietic stem cells, neuronal stem cells and mesenchymal stem cells.

Methods of delivering proteins comprising DNA-binding domains as described herein are described, for example, in U.S. Patent Nos. 6,453,242; 6,503,717; 6,534,261; 6,599,692; 6,607,882; 6,689,558; 6,824,978; 6,933,113; 6,979,539; 7,013,219; and 7,163,824, the disclosures of all of which are incorporated by reference herein in their entireties.

DNA binding domains and fusion proteins comprising these DNA binding domains as described herein may also be delivered using vectors containing sequences encoding one or more of the DNA-binding protein(s). Additionally, additional nucleic acids (*e*.*g*., donors) also may be delivered via these vectors. Any vector systems may be used including, but not limited to, plasmid vectors, retroviral vectors, lentiviral vectors, adenovirus vectors, poxvirus vectors; herpesvirus vectors and adeno-associated virus vectors, etc. See, also, U.S. Patent Nos. 6,534,261; 6,607,882; 6,824,978; 6,933,113; 6,979,539; 7,013,219; and 7,163,824, incorporated by reference herein in their entireties. Furthermore, it will be apparent that any of these vectors may comprise one or more DNA-binding protein-encoding sequences and/or additional nucleic acids as appropriate. Thus, when one or more DNA-binding proteins as described herein are introduced into the cell, and additional DNAs as appropriate, they may be carried on the same vector or on different vectors. When multiple vectors are used, each vector may comprise a sequence encoding one or multiple DNA-binding proteins and additional nucleic acids as desired.

Conventional viral and non-viral based gene transfer methods can be used to introduce nucleic acids encoding engineered DNA-binding proteins in cells (*e*.*g*., mammalian cells) and target tissues and to co-introduce additional nucleotide sequences as desired. Such methods can also be used to administer nucleic acids (*e.g.,* encoding DNA-binding proteins and/or donors) to cells *in vitro.* In certain embodiments, nucleic acids are administered for *in vivo* or *ex vivo* gene therapy uses. Non-viral vector delivery systems include DNA plasmids, naked nucleic acid, and nucleic acid complexed with a delivery vehicle such as a liposome or poloxamer. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology Doerfler and Böhm (eds.) (1995); and Yu et al., Gene Therapy 1:13-26 (1994).

Methods of non-viral delivery of nucleic acids include electroporation, lipofection, microinjection, biolistics, virosomes, liposomes, immunoliposomes, polycation or lipid:nucleic acid conjugates, naked DNA, mRNA, artificial virions, and agent-enhanced uptake of DNA. Sonoporation using, *e*.*g*., the Sonitron 2000 system (Rich-Mar) can also be used for delivery of nucleic acids. In a preferred embodiment, one or more nucleic acids are delivered as mRNA. Also preferred is the use of capped mRNAs to increase translational efficiency and/or mRNA stability. Especially preferred are ARCA (anti-reverse cap analog) caps or variants thereof. See U.S. Patent Nos. 7,074,596 and 8,153,773, incorporated by reference herein.

Additional exemplary nucleic acid delivery systems include those provided by Amaxa Biosystems (Cologne, Germany), Maxcyte, Inc. (Rockville, Maryland), BTX Molecular Delivery Systems (Holliston, MA) and Copernicus Therapeutics Inc, (see for example US6008336). Lipofection is described in *e.g.,* US 5,049,386, US 4,946,787; and US 4,897,355) and lipofection reagents are sold commercially (e.g., Transfectam^{™}, Lipofectin^{™}, and Lipofectamine^{™} RNAiMAX). Cationic and neutral lipids that are suitable for efficient receptor-recognition lipofection of polynucleotides include those of Felgner, WO 91/17424, WO 91/16024. Delivery can be to cells (*ex vivo* administration) or target tissues (*in vivo* administration).

The preparation of lipid:nucleic acid complexes, including targeted liposomes such as immunolipid complexes, is well known to one of skill in the art (see, *e.g.,* Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad etal., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

Additional methods of delivery include the use of packaging the nucleic acids to be delivered into EnGeneIC delivery vehicles (EDVs). These EDVs are specifically delivered to target tissues using bispecific antibodies where one arm of the antibody has specificity for the target tissue and the other has specificity for the EDV. The antibody brings the EDVs to the target cell surface and then the EDV is brought into the cell by endocytosis. Once in the cell, the contents are released (see MacDiarmid et al (2009) Nature Biotechnology 27(7) p. 643).

The use of RNA or DNA viral based systems for the delivery of nucleic acids encoding engineered DNA-binding proteins, and/or donors (*e*.*g*. CARs or ACTRs) as desired takes advantage of highly evolved processes for targeting a virus to specific cells in the body and trafficking the viral payload to the nucleus. Viral vectors can be administered directly to patients (*in vivo*) or they can be used to treat cells in vitro and the modified cells are administered to patients (*ex vivo*). Conventional viral based systems for the delivery of nucleic acids include, but are not limited to, retroviral, lentivirus, adenoviral, adeno-associated, vaccinia and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system depends on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immunodeficiency virus (SIV), human immunodeficiency virus (HIV), and combinations thereof (see, *e.g.,* Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991); PCT/US94/05700).

In applications in which transient expression is preferred, adenoviral based systems can be used. Adenoviral based vectors are capable of very high transduction efficiency in many cell types and do not require cell division. With such vectors, high titer and high levels of expression have been obtained. This vector can be produced in large quantities in a relatively simple system. Adeno-associated virus ("AAV") vectors are also used to transduce cells with target nucleic acids, *e*.*g*., in the *in vitro* production of nucleic acids and peptides, and for *in vivo* and *ex vivo* gene therapy procedures (see, *e.g.,* West et al., Virology 160:38-47 (1987); U.S. Patent No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS USA 81:6466-6470 (1984); and Samulski et al., J. Virol. 63:03822-3828 (1989).

At least six viral vector approaches are currently available for gene transfer in clinical trials, which utilize approaches that involve complementation of defective vectors by genes inserted into helper cell lines to generate the transducing agent.

pLASN and MFG-S are examples of retroviral vectors that have been used in clinical trials (Dunbar et al., Blood 85:3048-305 (1995); Kohn et al., Nat. Med. 1:1017-102 (1995); Malech et al., PNAS USA 94:22 12133-12138 (1997)). PA317/pLASN was the first therapeutic vector used in a gene therapy trial. (Blaese et al., Science 270:475-480 (1995)). Transduction efficiencies of 50% or greater have been observed for MFG-S packaged vectors. (Ellem et al., Immunol Immunother. 44(1):10-20 (1997); Dranoff et al., Hum. Gene Ther. 1:111-2 (1997).

Recombinant adeno-associated virus vectors (rAAV) are a promising alternative gene delivery system based on the defective and nonpathogenic parvovirus adeno-associated type 2 virus. All vectors are derived from a plasmid that retains only the AAV 145 bp inverted terminal repeats flanking the transgene expression cassette. Efficient gene transfer and stable transgene delivery due to integration into the genomes of the transduced cell are key features for this vector system. (Wagner et al., Lancet 351:9117 1702-3 (1998), Keams et al., Gene Ther. 9:748-55 (1996)). Other AAV serotypes, including AAV1, AAV3, AAV4, AAV5, AAV6, AAV8, AAV8.2, AAV9 and AAVrh10 and pseudotyped AAV such as AAV2/8, AAV2/5 and AAV2/6 can also be used in accordance with the present invention.

Replication-deficient recombinant adenoviral vectors (Ad) can be produced at high titer and readily infect a number of different cell types. Most adenovirus vectors are engineered such that a transgene replaces the Ad E1a, E1b, and/or E3 genes; subsequently the replication defective vector is propagated in human 293 cells that supply deleted gene function in *trans.* Ad vectors can transduce multiple types of tissues in vivo, including nondividing, differentiated cells such as those found in liver, kidney and muscle. Conventional Ad vectors have a large carrying capacity. An example of the use of an Ad vector in a clinical trial involved polynucleotide therapy for antitumor immunization with intramuscular injection (Sterman et al., Hum. Gene Ther. 7:1083-9 (1998)). Additional examples of the use of adenovirus vectors for gene transfer in clinical trials include Rosenecker et al., Infection 24:1 5-10 (1996); Sterman et al., Hum. Gene Ther. 9:7 1083-1089 (1998); Welsh et al., Hum. Gene Ther. 2:205-18 (1995); Alvarez et al., Hum. Gene Ther. 5:597-613 (1997); Topf et al., Gene Ther. 5:507-513 (1998); Sterman et al., Hum. Gene Ther. 7:1083-1089 (1998).

Packaging cells are used to form virus particles that are capable of infecting a host cell. Such cells include 293 cells, which package adenovirus, and ψ2 cells or PA317 cells, which package retrovirus. Viral vectors used in gene therapy are usually generated by a producer cell line that packages a nucleic acid vector into a viral particle. The vectors typically contain the minimal viral sequences required for packaging and subsequent integration into a host (if applicable), other viral sequences being replaced by an expression cassette encoding the protein to be expressed. The missing viral functions are supplied in *trans* by the packaging cell line. For example, AAV vectors used in gene therapy typically only possess inverted terminal repeat (ITR) sequences from the AAV genome which are required for packaging and integration into the host genome. Viral DNA is packaged in a cell line, which contains a helper plasmid encoding the other AAV genes, namely rep and cap, but lacking ITR sequences. The cell line is also infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, *e.g*., heat treatment to which adenovirus is more sensitive than AAV.

In many gene therapy applications, it is desirable that the gene therapy vector be delivered with a high degree of specificity to a particular tissue type. Accordingly, a viral vector can be modified to have specificity for a given cell type by expressing a ligand as a fusion protein with a viral coat protein on the outer surface of the virus. The ligand is chosen to have affinity for a receptor known to be present on the cell type of interest. For example, Han et al., (Proc. Natl. Acad. Sci. USA 92:9747-9751 (1995)), reported that Moloney murine leukemia virus can be modified to express human heregulin fused to gp70, and the recombinant virus infects certain human breast cancer cells expressing human epidermal growth factor receptor. This principle can be extended to other virus-target cell pairs, in which the target cell expresses a receptor and the virus expresses a fusion protein comprising a ligand for the cell-surface receptor. For example, filamentous phage can be engineered to display antibody fragments (*e.g*., FAB or Fv) having specific binding affinity for virtually any chosen cellular receptor. Although the above description applies primarily to viral vectors, the same principles can be applied to nonviral vectors. Such vectors can be engineered to contain specific uptake sequences which favor uptake by specific target cells.

Delivery methods for CRISPR/Cas systems can comprise those methods described above. For example, in animal models, *in vitro* transcribed Cas encoding mRNA or recombinant Cas protein can be directly injected into one-cell stage embryos using glass needles to genome-edited animals. To express Cas and guide RNAs in cells *in vitro*, typically plasmids that encode them are transfected into cells via lipofection or electroporation. Also, recombinant Cas protein can be complexed with *in vitro* transcribed guide RNA where the Cas-guide RNA ribonucleoprotein is taken up by the cells of interest (Kim et al (2014) Genome Res 24(6):1012). For therapeutic purposes, Cas and guide RNAs can be delivered by a combination of viral and non-viral techniques. For example, mRNA encoding Cas may be delivered via nanoparticle delivery while the guide RNAs and any desired transgene or repair template are delivered via AAV (Yin et al (2016) Nat Biotechnol 34(3) p. 328).

Gene therapy vectors can be delivered *in vivo* by administration to an individual patient, typically by systemic administration (e.g., intravenous, intraperitoneal, intramuscular, subdermal, or intracranial infusion) or topical application, as described below. Alternatively, vectors can be delivered to cells *ex vivo*, such as cells explanted from an individual patient (*e.g*., lymphocytes, bone marrow aspirates, tissue biopsy) or universal donor hematopoietic stem cells, followed by re-implantation of the cells into a patient, usually after selection for cells which have incorporated the vector.

*Ex vivo* cell transfection for diagnostics, research, transplant or for gene therapy (*e.g*., via re-infusion of the transfected cells into the host organism) is well known to those of skill in the art. In a preferred embodiment, cells are isolated from the subject organism, transfected with a DNA-binding proteins nucleic acid (gene or cDNA), and re-infused back into the subject organism (*e.g*., patient). Various cell types suitable for ex vivo transfection are well known to those of skill in the art (see, *e.g.*, Freshney et al., Culture of Animal Cells, A Manual of Basic Technique (3rd ed. 1994)) and the references cited therein for a discussion of how to isolate and culture cells from patients).

In one embodiment, stem cells are used in *ex vivo* procedures for cell transfection and gene therapy. The advantage to using stem cells is that they can be differentiated into other cell types in vitro, or can be introduced into a mammal (such as the donor of the cells) where they will engraft in the bone marrow. Methods for differentiating CD34+ cells in vitro into clinically important immune cell types using cytokines such a GM-CSF, IFN-γ and TNF-α are known (see Inaba et al., J. Exp. Med. 176:1693-1702 (1992)).

Stem cells are isolated for transduction and differentiation using known methods. For example, stem cells are isolated from bone marrow cells by panning the bone marrow cells with antibodies which bind unwanted cells, such as CD4+ and CD8+ (T cells), CD45+ (panB cells), GR-1 (granulocytes), and Iad (differentiated antigen presenting cells) (see Inaba et al., J. Exp. Med. 176:1693-1702 (1992)).

Stem cells that have been modified may also be used in some embodiments. For example, neuronal stem cells that have been made resistant to apoptosis may be used as therapeutic compositions where the stem cells also contain the ZFP TFs of the invention. Resistance to apoptosis may come about, for example, by knocking out BAX and/or BAK using BAX- or BAK-specific ZFNs (see, U.S. patent No. 8,597,912) in the stem cells, or those that are disrupted in a caspase, again using caspase-6 specific ZFNs for example.

Vectors (*e.g*., retroviruses, adenoviruses, liposomes, etc.) containing therapeutic DNA-binding proteins (or nucleic acids encoding these proteins) can also be administered directly to an organism for transduction of cells *in vivo.* Alternatively, naked DNA can be administered. Administration is by any of the routes normally used for introducing a molecule into ultimate contact with blood or tissue cells including, but not limited to, injection, infusion, topical application and electroporation. Suitable methods of administering such nucleic acids are available and well known to those of skill in the art, and, although more than one route can be used to administer a particular composition, a particular route can often provide a more immediate and more effective reaction than another route.

Methods for introduction of DNA into hematopoietic stem cells are disclosed, for example, in U.S. Patent No. 5,928,638. Vectors useful for introduction of transgenes into hematopoietic stem cells, *e.g*., CD34+ cells, include adenovirus Type 35.

Vectors suitable for introduction of transgenes into immune cells (*e.g*., T-cells) include non-integrating lentivirus vectors. See, for example, Ory et al. (1996) Proc. Natl. Acad. Sci. USA 93:11382-11388; Dull et al. (1998) J. Virol. 72:8463-8471; Zuffery et al. (1998) J. Virol. 72:9873-9880; Follenzi et al. (2000) Nature Genetics 25:217-222.

Pharmaceutically acceptable carriers are determined in part by the particular composition being administered, as well as by the particular method used to administer the composition. Accordingly, there is a wide variety of suitable formulations of pharmaceutical compositions available, as described below (see, *e.g*., Remington's Pharmaceutical Sciences, 17th ed., 1989).

As noted above, the disclosed methods and compositions can be used in any type of cell including, but not limited to, prokaryotic cells, fungal cells, Archaeal cells, plant cells, insect cells, animal cells, vertebrate cells, mammalian cells and human cells, including T-cells and stem cells of any type. Suitable cell lines for protein expression are known to those of skill in the art and include, but are not limited to COS, CHO (e.g., CHO-S, CHO-K1, CHO-DG44, CHO-DUXB11), VERO, MDCK, WI38, V79, B14AF28-G3, BHK, HaK, NS0, SP2/0-Ag14, HeLa, HEK293 (e.g., HEK293-F, HEK293-H, HEK293-T), perC6, insect cells such as *Spodoptera fugiperda* (Sf), and fungal cells such as Saccharomyces, Pichia and Schizosaccharomyces. Progeny, variants and derivatives of these cell lines can also be used.

### Applications

Use of engineered PD1 nucleases in treatment and prevention of disease, is expected to be useful in several fields. PD1 is involved in a variety of diseases and disorders, including but not limited to cancers and autoimmune diseases. *See, e.g*.*,* Chamoto et al. (2017) Curr Top Microbiol Immunol. 410:75-97. For instance, PD1 has been shown to be involved in regulating the balance between T cell activation and T cell tolerance in response to chronic antigens. During HIV1 infection, expression of PD1 has been found to be increased in CD4+ T cells. It is thought that PD1 up-regulation is somehow tied to T cell exhaustion (defined as a progressive loss of key effector functions) when T cell dysfunction is observed in the presence of chronic antigen exposure as is the case in HIV infection. PD1 up-regulation may also be associated with increased apoptosis in these same sets of cells during chronic viral infection (*see* Petrovas et al, (2009) J Immunol. 183(2):1120-32). PD1 may also play a role in tumor-specific escape from immune surveillance. It has been demonstrated that PD1 is highly expressed in tumor-specific cytotoxic T lymphocytes (CTLs) in both chronic myelogenous leukemia (CML) and acute myelogenous leukemia (AML). PD1 is also up-regulated in melanoma infiltrating T lymphocytes (TILs) (*see* Dotti (2009) Blood 114 (8): 1457-58). Tumors have been found to express the PD1 ligand (PDL) which, when combined with the up-regulation of PD1 in CTLs, may be a contributory factor in the loss in T cell functionality and the inability of CTLs to mediate an effective anti-tumor response. Thus, the methods and compositions described herein serve to increase the specificity of these novel tools to ensure that the desired PD1 target sites (*e.g*., for treatment of disorders such as cancers and autoimmune disorders) will be the primary (or only) place of cleavage. Minimizing or eliminating off-target cleavage as described herein increases the potential of this technology, for all *in vitro*, *in vivo* and *ex vivo* applications.

Nuclease-mediated genetic modification of PD1 is useful in treatment of a variety of genetic and other diseases, including but not limited to cancers. For example, genetically modified T cells (*e.g.*, CAR+ cells) can be further modified by genetic modification of PD1 as described herein to provide therapeutic compositions for a cancer.

As noted above, the compositions and methods described herein can be used for gene modification, gene correction, and gene disruption. Non-limiting examples of gene modification includes homology directed repair (HDR)-based targeted integration; HDR-based gene correction; HDR-based gene modification; HDR-based gene disruption; NHEJ-based gene disruption and/or combinations of HDR, NHEJ, and/or single strand annealing (SSA). Single-Strand Annealing (SSA) refers to the repair of a double strand break between two repeated sequences that occur in the same orientation by resection of the DSB by 5'-3' exonucleases to expose the 2 complementary regions. The single-strands encoding the 2 direct repeats then anneal to each other, and the annealed intermediate can be processed such that the single-stranded tails (the portion of the single-stranded DNA that is not annealed to any sequence) are be digested away, the gaps filled in by DNA Polymerase, and the DNA ends rejoined. This results in the deletion of sequences located between the direct repeats.

The compositions and methods can also be used for somatic cell therapy, thereby allowing production of stocks of cells that have been modified to enhance their biological properties. Such cells can be infused into a variety of patients, independent of the donor source of the cells and their histocompatibility to the recipient.

The engineered cleavage half domains described can also be used in gene modification protocols requiring simultaneous cleavage at multiple targets either to delete the intervening region or to alter two specific loci at once. Cleavage at two targets would require cellular expression of four ZFNs or TALENs, which could yield potentially ten different active ZFN or TALEN combinations. For such applications, substitution of these novel variants for the wild-type nuclease domain would eliminate the activity of the undesired combinations and reduce chances of off-target cleavage. If cleavage at a certain desired DNA target requires the activity of the nuclease pair A+B, and simultaneous cleavage at a second desired DNA target requires the activity of the nuclease pair X+Y, then use of the mutations described herein can prevent the pairings of A with A, A with X, A with Y and so on.

### Exemplary References Cited

Beane JD, Lee G, Zheng Z, Mendel M, Abate-Daga D, Bharathan M, Black M, Gandhi N, Yu Z, Chandran S, Giedlin M, Ando D, Miller J, Paschon D, Guschin D, Rebar EJ, Reik A, Holmes MC, Gregory PD, Restifo NP, Rosenberg SA, Morgan RA, Feldman SA (2015). Clinical Scale Zinc Finger Nuclease-mediated Gene Editing of PD-1 in Tumor Infiltrating Lymphocytes for the Treatment of Metastatic Melanoma. Mol Ther. 23:1380-1390. doi: 10.1038/mt.2015.71.
Bibikova M, Beumer K, Trautman JK , and Carroll, D (2003). Enhancing gene targeting with designed zinc finger nucleases. Science, 300:764.
Bibikova M, Golic M, Golic KG and Carroll D (2002). Targeted chromosomal cleavage and mutagenesis in Drosophila using zinc-finger nucleases. Genetics, 161:1169-1175.
Briggs GE, Haldane JBS (1925). A Note on the Kinetics of Enzyme Action. Biochem J. 19: 338-339.
Bolukbasi MF, Gupta A, Oikemus S, Derr AG, Garber M, Brodsky MH, Zhu LJ, Wolfe SA (2015). DNA-binding-domain fusions enhance the targeting range and precision of Cas9. Nat Methods. 12:1150-6. doi: 10.1038/nmeth.3624.
Casini A, Olivieri M, Petris G, Montagna C, Reginato G, Maule G, Lorenzin F, Prandi D, Romanel A, Demichelis F, Inga A, Cereseto A (2018). A highly specific SpCas9 variant is identified by in vivo screening in yeast. Nat Biotechnol. 36:265-271. doi: 10.1038/nbt.4066.
Chen JS, Dagdas YS, Kleinstiver BP, Welch MM, Sousa AA, Harrington LB, Sternberg SH, Joung JK, Yildiz A, Doudna JA (2017). Enhanced proofreading governs CRISPR-Cas9 targeting accuracy. Nature. 550:407-410. doi: 10.1038/nature24268.
Dagdas YS, Chen JS, Sternberg SH, Doudna JA, Yildiz A (2017). A conformational checkpoint between DNA binding and cleavage by CRISPR-Cas9. Sci Adv. 3:eaao0027. doi: 10.1126/sciadv.aao0027.
De Ravin SS, Reik A, Liu PQ, Li L, Wu X, Su L, Raley C, Theobald N, Choi U, Song AH, Chan A, Pearl JR, Paschon DE, Lee J, Newcombe H, Koontz S, Sweeney C, Shivak DA, Zarember KA, Peshwa MV, Gregory PD, Umov FD, Malech HL (2016). Targeted gene addition in human CD34(+) hematopoietic cells for correction of X-linked chronic granulomatous disease. Nat Biotechnol. 34:424-9. doi: 10.1038/nbt.3513.
DeWitt MA, Magis W, Bray NL, Wang T, Berman JR, Urbinati F, Heo SJ, Mitros T, Muñoz DP, Boffelli D, Kohn DB, Walters MC, Carroll D, Martin DI, Corn JE (2016). Selection-free genome editing of the sickle mutation in human adult hematopoietic stem/progenitor cells. Sci Transl Med. 8:360ra134.
DiCarlo JE, Mahajan VB, Tsang SH (2018). Gene therapy and genome surgery in the retina. J Clin Invest. 128:2177-2188. doi: 10.1172/JCI120429.
Donsante A, Miller DG, Li Y, Vogler C, Brunt EM, Russell DW, Sands MS (2007). AAV vector integration sites in mouse hepatocellular carcinoma. Science. 317:477. Doyon Y, Vo TD, Mendel MC, Greenberg SG, Wang J, Xia DF, Miller JC, Umov FD, Gregory PD, Holmes MC (2011). Enhancing zinc-finger-nuclease activity with improved obligate heterodimeric architectures. Nat Methods. 8:74-9. doi: 10.1038/nmeth.1539.
Fersht AR, Dingwall C (1979). Evidence for the double-sieve editing mechanism in protein synthesis. Steric exclusion of isoleucine by valyl-tRNA synthetases. Biochemistry. 18(12):2627-31.
Gao X, Tao Y, Lamas V, Huang M, Yeh WH, Pan B, Hu YJ, Hu JH, Thompson DB, Shu Y, Li Y, Wang H, Yang S, Xu Q, Polley DB, Liberman MC, Kong WJ, Holt JR, Chen ZY, Liu DR (2018). Treatment of autosomal dominant hearing loss by in vivo delivery of genome editing agents. Nature. 553(7687):217-221. doi: 10.1038/nature25164.
Grizot S, Smith J, Daboussi F, Prieto J, Redondo P, Merino N, Villate M, Thomas S, Lemaire L, Montoya G, Blanco FJ, Paques F, Duchateau P (2009). Efficient targeting of a SCID gene by an engineered single-chain homing endonuclease. Nucleic Acids Res. 37:5405-19. doi: 10.1093/nar/gkp548.
Guilinger JP, David B Thompson & David R Liu (2014a). Fusion of catalytically inactive Cas9 to FokI nuclease improves the specificity of genome modification. Nature Biotechnol. 32:577-582.
Guilinger JP, Pattanayak V, Reyon D, Tsai SQ, Sander JD, Joung JK, Liu DR (2014b). Broad specificity profiling of TALENs results in engineered nucleases with improved DNA-cleavage specificity.Nat Methods. 11:429-35. doi: 10.1038/nmeth.2845.
Hopfield JJ (1974). Kinetic proofreading: a new mechanism for reducing errors in biosynthetic processes requiring high specificity. Proc. Natl. Acad. Sci. U.S.A. 71: 4135-9.
Hu JH, Miller SM1,2,3, Geurts MH1,2,3, Tang W1,2,3, Chen L1,2,3, Sun N1,2,3, Zeina CM1,2,3, Gao X1,2,3, Rees HA1,2,3, Lin Z1,2,3, Liu DR (2018). Evolved Cas9 variants with broad PAM compatibility and high DNA specificity. Nature. 556:57-63. doi: 10.1038/nature26155.
Huang CH, Lee KC, Doudna JA (2018). Applications of CRISPR-Cas Enzymes in Cancer Therapeutics and Detection. Trends Cancer. 2018 4:499-512. doi: 10.1016/j.trecan.2018.05.006.
Kim JS, Pabo CO (1998). Getting a handhold on DNA: design of poly-zinc finger proteins with femtomolar dissociation constants. Proc Natl Acad Sci U S A. 95:2812-7.
Kleinstiver BP, Pattanayak V, Prew MS, Tsai SQ, Nguyen NT, Zheng Z, Joung JK (2016). High-fidelity CRISPR-Cas9 nucleases with no detectable genome-wide off-target effects. Nature. 529:490-5. doi: 10.1038/nature16526..
Jarjour J, West-Foyle H, Certo MT, Hubert CG, Doyle L, Getz MM, Stoddard BL, Scharenberg AM (2009). High-resolution profiling of homing endonuclease binding and catalytic specificity using yeast surface display. Nucleic Acids Res. 37:6871-80. doi: 10.1093/narlgkp726.
Jiang F, Taylor DW, Chen JS, Kornfeld JE, Zhou K, Thompson AJ, Nogales E, Doudna JA (2016). Structures of a CRISPR-Cas9 R-loop complex primed for DNA cleavage. Science. 351:867-71. doi: 10.1126/science.aad8282.
Jiang F, Doudna JA (2017). CRISPR-Cas9 Structures and Mechanisms. Annu Rev Biophys. 46:505-529. doi: 10.1146/annurev-biophys-062215-010822.
Kim YG, Cha J, Chandrasegaran S (1996). Hybrid restriction enzymes: zinc finger fusions to FokI cleavage domain. Proc Natl Acad Sci U S A. 93:1156-60
Kulcsár PI, Tálas A, Huszár K, Ligeti Z, Tóth E, Weinhardt N, Fodor E, Welker E (2017). Crossing enhanced and high fidelity SpCas9 nucleases to optimize specificity and cleavage. Genome Biol. 18:190. doi: 10.1186/s13059-017-1318-8.
Michaelis L, Menten ML (1913). Die Kinetik der Invertinwirkung, Biochemische Zeitschrift 49: 333-369
Miller JC, Holmes MC, Wang J, Guschin DY, Lee YL, Rupniewski I, Beausejour CM, Waite AJ, Wang NS, Kim KA, Gregory PD, Pabo CO, Rebar EJ (2007). An improved zinc-finger nuclease architecture for highly specific genome editing. Nat Biotechnol. 25:778-85. DOI: 10.1038/nbt1319
Miller JC, Zhang L, Xia DF, Campo JJ, Ankoudinova IV, Guschin DY, Babiarz JE, Meng X, Hinkley SJ, Lam SC, Paschon DE, Vincent AI, Dulay GP, Barlow KA, Shivak DA, Leung E, Kim JD, Amora R, Umov FD, Gregory PD, Rebar EJ (2015). Improved specificity of TALE-based genome editing using an expanded RVD repertoire. Nat Methods. 12:465-71. doi: 10.10381nmeth.3330.
Miller JC, Tan S, Qiao G, Barlow KA, Wang J, Xia DF, Meng X, Paschon DE, Leung E, Hinkley SJ, Dulay GP, Hua KL, Ankoudinova I, Cost GJ, Urnov FD, Zhang HS, Holmes MC, Zhang L, Gregory PD, Rebar EJ (2011). A TALE nuclease architecture for efficient genome editing. Nat Biotechnol. 29:143-8. doi: 10.1038/nbt.1755.
Pattanayak V, Ramirez CL, Joung JK, Liu, DR (2011). Revealing off-target cleavage specificities of zinc-finger nucleases by in vitro selection. Nature methods, 8:765-770.
Pavletich NP, Pabo CO (1991). Zinc finger-DNA recognition: crystal structure of a Zif268-DNA complex at 2.1 A. Science. 252:809-17.
Pernstich C, Halford SE (2012). Illuminating the reaction pathway of the FokI restriction endonuclease by fluorescence resonance energy transfer. Nucleic Acids Res. 40:1203-13. doi: 10.1093/nar/gkr809.
Porteus MH, Baltimore D (2003). Chimeric Nucleases Stimulate Gene Targeting in Human Cells. Science 300:763 DOI: 10.1126/science.1078395
Ran FA, Cong L, Yan WX, Scott DA, Gootenberg JS, Kriz AJ, Zetsche B, Shalem O, Wu X, Makarova KS, Koonin EV, Sharp PA, Zhang F (2015). In vivo genome editing using Staphylococcus aureus Cas9. Nature. 520:186-91. doi: 10.1038/nature14299.
Ran FA, Hsu PD, Lin CY, Gootenberg JS, Konermann S, Trevino AE, Scott DA, Inoue A, Matoba S, Zhang Y, Zhang F (2013). Double nicking by RNA-guided CRISPR Cas9 for enhanced genome editing specificity. Cell. 154:1380-9. doi: 10.1016/j.cell.2013.08.021.
Raper AT, Anthony A. Stephenson, Suo Z (2018). Functional Insights Revealed by the Kinetic Mechanism of CRISPR/Cas9 J. Am. Chem. Soc. 140:2971-2984 DOI: 10.1021/jacs.7b13047
Ryan DE, Taussig D, Steinfeld I, Phadnis SM, Lunstad BD, Singh M, Vuong X, Okochi KD, McCaffrey R, Olesiak M, Roy S, Yung CW, Curry B, Sampson JR, Bruhn L, Dellinger DJ (2018). Improving CRISPR-Cas specificity with chemical modifications in single-guide RNAs. Nucleic Acids Res. 46:792-803. doi: 10.1093/nar/gkx1199.
Seki A and Rutz S (2018). Optimized RNP transfection for highly efficient CRISPR/Cas9-mediated gene knockout in primary T cells. J Exp Med. 215: 985-997. doi: 10.1084/jem.20171626
Singh D, Sternberg SH, Fei J, Doudna JA, Ha T (2016). Real-time observation of DNA recognition and rejection by the RNA-guided endonuclease Cas9. Nat Commun. 7:12778. doi: 10.1038/ncomms12778.
Slaymaker IM, Gao L, Zetsche B, Scott DA, Yan WX, Zhang F (2016). Rationally engineered Cas9 nucleases with improved specificity. Science. 351:84-8. doi: 10.1126/science.aad5227.
Sternberg SH, LaFrance B, Kaplan M, Doudna JA (2015). Conformational control of DNA target cleavage by CRISPR-Cas9. Nature. 527:110-3. doi: 10.1038/nature15544.
Tsai SQ, Nicolas Wyvekens, Cyd Khayter, Jennifer A Foden, Vishal Thapar, Deepak Reyon, Mathew J Goodwin, Martin J Aryee ,Joung JK (2014). Dimeric CRISPR RNA-guided FokI nucleases for highly specific genome editing. Nature Biotechnol. 32:569-576
Tsai SQ, Zheng Z, Nguyen NT, Liebers M, Topkar VV, Thapar V, Wyvekens N, Khayter C, Iafrate AJ, Le LP, Aryee MJ, Joung JK (2015). GUIDE-seq enables genome-wide profiling of off-target cleavage by CRISPR-Cas nucleases. Nat Biotechnol. 33:187-197. doi: 10.1038/nbt.3117.
Tsai SQ and Joung JK (2016). Defining and improving the genome-wide specificities of CRISPR-Cas9 nucleases. Nature Reviews Genetics. 17, 300-312.
Umov FD, Miller, JC, Lee YL, Beausejour CM, Rock JM, Augustus S, Jamieson AC, Porteus MH, Gregory PD and Holmes MC (2005). Highly efficient endogenous human gene correction using designed zinc-finger nucleases. Nature, 435, 646-651.
Yin H, Song CQ, Suresh S, Kwan SY, Wu Q, Walsh S, Ding J, Bogorad RL, Zhu LJ, Wolfe SA, Koteliansky V, Xue W, Langer R, Anderson DG (2018). Partial DNA-guided Cas9 enables genome editing with reduced off-target activity. Nat Chem Biol. 14:311-316. doi: 10.1038/nchembio.2559.
Zhang D, Zhang H, Li T, Chen K, Qiu JL, Gao C (2017). Perfectly matched 20-nucleotide guide RNA sequences enable robust genome editing using high-fidelity SpCas9 nucleases. Genome Biol. 18:191. doi: 10.1186/s13059-017-1325-9.

### EXAMPLES

### Example 1: Generation of Specific PD1-specific Nucleases

PD1-specific nucleases with low levels of off-target effects were generated as described in U.S. Patent Publication No. 20180087072. The original (parent) ZFNs comprise ZFPs 12942 and 25029, as described in U.S. Patent No. 8,563,314 (*e.g*., Tables 2 and 3).

The nucleotide and amino acid sequences of the parent ZFN pair is shown below (recognition helix regions underlined in amino acid sequence, *Fok*I residues targeted shown in lower case, *Fok*I positions chosen for analysis are in bold underline):
12942 (Left PD1 ZFN)
   Nucleic acid sequence: 
ATGGACTACAAAGACCATGACGGTGATTATAAAGATCATGACATCGATTA
CAAGGATGACGATGACAAGATGGCCCCCAAGAAGAAGAGGAAGGTGGGC
ATTCACGGGGTACCCGCCGCTATGGCTGAGAGGCCCTTCCAGTGTCGAATCTGCAT
GCGTAAGTTTGCCCAGTCCGGCCACCTGTCCCGCCATACCAAGATACACACGGGCGA
GAAGCCCTTCCAGTGTCGAATCTGCATGCGTAACTTCAGTCGTAGTGACAGCCTGAG
CGTACACATCCGCACCCACACAGGCGAGAAGCCTTTTGCCTGTGACATTTGTGGGAG
GAAATTTGCCCACAACGACAGCCGCAAAAACCATACCAAGATACACACGGGATCTCA
GAAGCCCTTCCAGTGTCGAATCTGCATGCGTAACTTCAGTCGCTCCGACGACCTGAC
CCGCCACATCCGCACCCACACAGGCGAGAAGCCTTTTGCCTGTGACATTTGTGGGAG
GAAGTTTGCCCGCTCCGACCACCTGACCCAGCATACCAAGATACACCTGCGGGGATC
CCAGCTGGTGAAGAGCGAGCTGGAGGAGAAGAAGTCCGAGCTGCGGCAC
AAGCTGAAGTACGTGCCCCACGAGTACATCGAGCTGATCGAGATCGCCAG
GAACAGCACCCAGGACCGCATCCTGGAGATGAAGGTGATGGAGTTCTTCA
TGAAGGTGTACGGCTACAGGGGAAAGCACCTGGGCGGAAGCAGAAAGCC
TGACGGCGCCATCTATACAGTGGGCAGCCCCATCGATTACGGCGTGATCG
TGGACACAAAGGCCTACAGCGGCGGCTACAATCTGCCTATCGGCCAGGCC
GACGAGATGCAGAGATACGTGGAGGAGAACCAGACCCGGAATAAGCACA
TCAACCCCAACGAGTGGTGGAAGGTGTACCCTAGCAGCGTGACCGAGTTC
AAGTTCCTGTTCGTGAGCGGCCACTTCAAGGGCAACTACAAGGCCCAGCT
GACCAGGCTGAACCACATCACCAACTGCAATGGCGCCGTGCTGAGCGTGG
AGGAGCTGCTGATCGGCGGCGAGATGATCAAAGCCGGCACCCTGACACTG
GAGGAGGTGCGGCGCAAGTTCAACAACGGCGAGATCAACTTCAGATCTTG
ATAA (SEQ ID NO: 2).
Amino acid sequence: 
MDYKDHDGDYKDHDIDYKDDDDKMAPKKKRKVGIHGVPAAMAERPFQCRIC
MRKFAQSGHLSRHTKIHTGEKPFQCRICMRNFSRSDSLSVHIRTHTGEKPFACDICG
RKFAHNDSRKNHTKIHTGSQKPFQCRICMRNFSRSDDLTRHIRTHTGEKPFACDICG
RKFARSDHLTQHTKIHLRGSQLVKSELEEKKSELRHKLKYVPHEYIELIEIARNS
TQDRILEMKVMEFFMKVYGYRGKHLGGSRKPDGAIYTVGSPIDYGVIVDTK
AYSGGYNLPIGQADEMQRYVEENQTRNKHINPNEWWKVYPSSVTEFKFLFV
SGHFKGNYKAQLTRLNHITNCNGAVLSVEELLIGGEMIKAGTLTLEEVRRKF
NNGEINFRS(SEQ ID NO:3).
25029 (Right PD1 ZFN)
   Nucleic acid sequence: 
ATGGACTACAAAGACCATGACGGTGATTATAAAGATCATGACATCGATTA
CAAGGATGACGATGACAAGATGGCCCCCAAGAAGAAGAGGAAGGTGGGC
ATTCACGGGGTACCCGCCGCTATGGCTGAGAGGCCCTTCCAGTGTCGAATCTGCAT
GTGTAAGTTTGCCCGCAACGCCGCCCTGACCCGCCATACCAAGATACACACGGGCGA
GAAGCCGTTCCAGTGTCGCATCTGCATGCGTAACTTCAGTCGCTCCGACGAGCTGAC
CCGCCACATCCGCACCCACACAGGCGAGAAGCCTTTTGCTTGCGACATTTGTGGGAG
GAAGTTTGCCCGGCACCACCACCTGGCCGCCCATACCAAGATACACACGGGATCTCA
GAAGCCCTTCCAGTGTCGAATCTGCATGCGTAACTTCAGTACCCGCCCGGTGCTGAA
GCGCCACATCCGCACCCACACAGGCGAGAAGCCTTTTGCTTGCGACATTTGTGGGAG
GAAGTTTGCCGACCGCTCCGCCCTGGCCCGCCATACCAAGATACACCTGCGGGGATC
CCAGCTGGTGAAGAGCGAGCTGGAGGAGAAGAAGTCCGAGCTGCGGCAC
AAGCTGAAGTACGTGCCCCACGAGTACATCGAGCTGATCGAGATCGCCAG
GAACAGCACCCAGGACCGCATCCTGGAGATGAAGGTGATGGAGTTCTTCA
TGAAGGTGTACGGCTACAGGGGAAAGCACCTGGGCGGAAGCAGAAAGCC
TGACGGCGCCATCTATACAGTGGGCAGCCCCATCGATTACGGCGTGATCG
TGGACACAAAGGCCTACAGCGGCGGCTACAATCTGCCTATCGGCCAGGCC
GACGAGATGCAGAGATACGTGGAGGAGAACCAGACCCGGAATAAGCACA
TCAACCCCAACGAGTGGTGGAAGGTGTACCCTAGCAGCGTGACCGAGTTC
AAGTTCCTGTTCGTGAGCGGCCACTTCAAGGGCAACTACAAGGCCCAGCT
GACCAGGCTGAACCACATCACCAACTGCAATGGCGCCGTGCTGAGCGTGG
AGGAGCTGCTGATCGGCGGCGAGATGATCAAAGCCGGCACCCTGACACTG
GAGGAGGTGCGGCGCAAGTTCAACAACGGCGAGATCAACTTCAGATCTTG
ATAA (SEQ ID NO:4)
Amino acid sequence:

MDYKDHDGDYKDHDIDYKDDDDKMAPKKKRKVGIHGVPAAMAERPFQCRIC
MCKFARNAALTRHTKIHTGEKPFQCRICMRNFSRSDELTRHIRTHTGEKPFACDICG
RKFARHHHLAAHTKIHTGSQKPFQCRICMRNFSTRPVLKRHIRTHTGEKPFACDICG
RKFADRSALARHTKIHLRGSQLVKSELEEKKSELRHKLKYVPHEYIELIEIARNS
TQDRILEMKVMEFFMKVYGYRGKHLGGSRKPDGAIYTVGSPIDYGVIVDTK
AYSGGYNLPIGQADEMQRYVEENQTRNKHINPNEWWKVYPSSVTEFKFLFV
SGHFKGNYKAQLTRLNHITNCNGAVLSVEELLIGGEMIKAGTLTLEEVRRKF
NNGEINFRS (SEQ ID NO: 5).

Using these PD1 ZFNs, a panel of 22 *FokI* variants (listed in Figure 1, including R416E, R416F, R416N, S418D, S418E, R422H, N476D, K448A, N476E, N476G, N476T, I479T, I479Q, Q481A, Q481D, Q481E, Q481H, K525A, K525S, K525T, K525V, N527D, Q531R) was screened. On-target activity of variants of the PD1 ZFN dimer bearing single-residue substitutions within their *Fok*I domain was analyzed. Each variant was tested as a dimer in which both ZFNs bore the indicated substitution ZFNs and were delivered to human K562 cells via mRNA nucleofection (500 ng of each monomer), followed by genomic DNA isolation at day 3 and deep sequencing analysis for indels at the intended target. "Half dose" parent samples used 250 ng RNA for delivery. To highlight relative signal intensities, table values are shaded. Arrows highlight exemplary variants manifesting full retention of high levels of on-target activity.

The *Fok*I variants identified from the on-target screening, including R416E, R416F, R416N, R422H, N476G, N476T, Q481D, Q481H, K525A, K525S, K525T and K525V) were further characterized for activity at both and on target and off-target sites, including the 3 most highly modified previously described off-targets (Beane 2015). In brief, ZFNs with the same *Fok*I variant in both ZFNs of the pair were delivered to human K562 cells via nucleofection using the indicated amount of mRNA for each ZFN monomer. Genomic DNA was isolated at 3 days and deep sequencing analysis for indels at the intended target and at off-target sites were determined.

As shown in Figure 2A through Figure 3, off-target activity was substantially reduced (10 to 100 fold), with the most selective substitution (Q481D) manifesting a >200 fold increase in on-target cleavage preference (*see*, Figure 3 in which 75% PD1 indels / 6.4% OT indels for the parent dimer, vs 81% / 0.03% for the variant).

As shown herein, PD1 ZFNs were generated with highly enhanced specificity via modification of the *Fok*I domains.

### Example 2: TALEN FokI Variants

TALEN pairs were designed and tested as described in U.S. Patent Publication No. 20180087072. The TALENs pairs ("sample") used are all *Fok*I variants of the TAL-effectors comprising the DNA binding domains of 101041 and 101047 as described in U.S. Patent No. 8,586,526, which were renamed as shown in Figure 4 based on the *Fok*I variant included.

As shown in Figure 4, several FokI variant constructs were more active, including TALENs including the S446R mutation, the Q5531R or the Q481H mutation (in one or both TALENs of the dimer). At 30°C, using the *Fok*I variant Q531R on the right TALEN and using the *Fok*I variant Q481H on both the left and right TALENs decreased off-target activity by more than 4-fold while retaining full on-target activity. Furthermore, most TALENs exhibited an at least 2 to 8-fold increase in specificity (as measured by relative on/off levels).

All patents, patent applications and publications mentioned herein are hereby incorporated by reference in their entirety.

Although disclosure has been provided in some detail by way of illustration and example for the purposes of clarity of understanding, it will be apparent to those skilled in the art that various changes and modifications can be practiced without departing from the spirit or scope of the disclosure. Accordingly, the foregoing descriptions and examples should not be construed as limiting.

### Additional embodiments of the invention:

1. A zinc finger nuclease (ZFN) or TALEN that cleaves a programmed cell death 1 (PD1) gene, the ZFN or TALEN comprising first and second ZFNs and TALENs, each ZFN comprising a ZFP DNA-binding domain that binds to a target site in the PD1 gene and a *Fok*I cleavage domain, each TALEN comprising a TAL-effector DNA-binding domain that binds to a target site in the PD1 gene and a *Fok*I cleavage domain, wherein at least one of the *FokI* cleavage domains of the ZFN or TALEN further comprises a substitution mutation in the *Fok*I cleavage domain at one or more of 416, 418, 422, 476, 479, 481, 525, 527 or 531, numbered relative to wild-type *Fok*I.
2. The ZFN or TALEN of embodiment 1, wherein the substitution mutation in the at least one *Fok*I cleavage domain is as follows: R416E, R416F, R416N, S418D, S418E, R422H, N476D, N476E, N476G, N476T, I479T, I479Q, Q481A, Q481D, Q481E, Q481H, K525A, K525S, K525T, K525V, N527D and/or Q531R.
3. The ZFN or TALEN of any of the preceding embodiments, wherein the substitution mutation in the *Fok*I cleavage domain is as follows: R416E, R416F, R416N, R422H, N476G, N476T, Q481D, Q481H, K525A, K525S, K525T or K525V.
4. The ZFN or TALEN of any of the preceding embodiments wherein the first and/or the second ZFN or TALENs comprise the substitution mutation.
5. The ZFN of any of the preceding embodiments, wherein the first ZFP DNA-binding domain comprises the ZFP designated 12942 having the amino acid sequence as shown in SEQ ID NO:3 and the second ZFP DNA-binding domain comprises the ZFP designated 25029 having the amino acid sequence as shown in SEQ ID NO:5.
6. One or more polynucleotides encoding the ZFN or TALEN according to any of the preceding embodiments.
7. An isolated cell comprising the one or more ZFNs, one or more TALENs and/or one or more polynucleotides of any of the preceding embodiments.
8. A method for cleaving a PD1 gene in a mammalian cell, the method comprising:
   expressing one or more polynucleotides encoding one or more ZFNs or TALENs of any of the preceding embodiments in the cell such that the PD 1 gene is cleaved.
9. The method of embodiment 8, further comprising contacting the cell with a donor polynucleotide; wherein cleavage of the PD 1 gene facilitates homologous recombination between the donor polypeptide and the PD1 gene.
10. An isolated population of genetically modified cells produced from the isolated cell or methods of any of the preceding embodiments, wherein PD1 gene is specifically modified by the nucleases.
11. The isolated population of genetically modified cells, wherein the on target to off-target ratio of genetic modification of PD1 is greater than 200.
12. A partially or fully differentiated cell descended from the isolated population of genetically modified cells of any of the preceding embodiments.
13. The isolated population of genetically modified cells of any of the preceding embodiments, further comprising one or more additional genetic modifications, optionally comprising inactivating one or more genes other than PD1 such as a T cell receptor gene, a B2M gene and/or a CTLA-4 gene, and/or integration of a transgene such as a CAR transgene.
14. A composition comprising one or more ZFNs or TALENs, one or more polynucleotides or the isolated population of cells according to any of the preceding embodiments for use in treatment of a cancer.
15. A method of treating a disease or disorder in a subject, the method comprising cleaving a PD1 gene administering one or more ZFNs or TALENs, one or more polynucleotides or the isolated population of cells according to any of the preceding embodiments to a subject in need thereof.
16. The method of embodiment 15, wherein the disease or disorder is a cancer or an autoimmune disorder.

## Claims

1. A zinc finger nuclease (ZFN) or TALEN that cleaves a programmed cell death 1 (PD1) gene, the ZFN or TALEN comprising first and second ZFNs and TALENs, each ZFN comprising a ZFP DNA-binding domain that binds to a target site in the PD1 gene and a *Fok*I cleavage domain, each TALEN comprising a TAL-effector DNA-binding domain that binds to a target site in the PD1 gene and a *Fok*I cleavage domain, wherein at least one of the *FokI* cleavage domains of the ZFN or TALEN further comprises a substitution mutation in the *Fok*I cleavage domain at one or more of 416, 422, 476, or 525, numbered relative to wild-type *Fok*I, wherein the one or more substitution mutations provide increased specificity.

2. The ZFN or TALEN of claim 1, wherein the substitution mutation in the at least one *Fok*I cleavage domain is as follows: R416E, R416F, R416N, R422H, N476D, N476E, N476G, N476T, K525A, K525S, K525T, and/or K525V.

3. The ZFN or TALEN of any of the preceding claims, wherein the substitution mutation in the *Fok*I cleavage domain is as follows: R416E, R416F, R416N, R422H, N476G, N476T, K525A, K525S, K525T or K525V.

4. The ZFN or TALEN of any of the preceding claims wherein the first and/or the second ZFN or TALENs comprise the substitution mutation.

5. The ZFN of any of the preceding claims, wherein the first ZFP DNA-binding domain comprises the ZFP designated 12942 having the amino acid sequence as shown in SEQ ID NO:3 and the second ZFP DNA-binding domain comprises the ZFP designated 25029 having the amino acid sequence as shown in SEQ ID NO:5.

6. One or more polynucleotides encoding the ZFN or TALEN according to any of the preceding claims.

7. An isolated cell comprising the one or more ZFNs, one or more TALENs and/or one or more polynucleotides of any of the preceding claims.

8. A method for cleaving a PD1 gene in a mammalian cell, the method comprising:
expressing one or more polynucleotides encoding one or more ZFNs or TALENs of any of the preceding claims in the cell such that the PD 1 gene is cleaved.

9. The method of claim 8, further comprising contacting the cell with a donor polynucleotide; wherein cleavage of the PD 1 gene facilitates homologous recombination between the donor polypeptide and the PD1 gene.

10. An isolated population of genetically modified cells produced from the isolated cell or methods of any of the preceding claims, wherein PD1 gene is specifically modified by the nucleases.

11. The isolated population of genetically modified cells of claim 10,
wherein the on target to off-target ratio of genetic modification of PD1 is greater than 200.

12. A partially or fully differentiated cell descended from the isolated population of genetically modified cells of any of the preceding claims.

13. The isolated population of genetically modified cells of any of the preceding claims, further comprising one or more additional genetic modifications, optionally comprising inactivating one or more genes other than PD1 such as a T cell receptor gene, a B2M gene and/or a CTLA-4 gene, and/or integration of a transgene such as a CAR transgene.

14. A composition comprising one or more ZFNs or TALENs, one or more polynucleotides or the isolated population of cells according to any of the preceding claims for use in treatment of a cancer.

15. A method of treating a disease or disorder in a subject, the method comprising cleaving a PD1 gene administering one or more ZFNs or TALENs, one or more polynucleotides or the isolated population of cells according to any of the preceding claims to a subject in need thereof, optionally wherein the disease or disorder is a cancer or an autoimmune disorder.
